(19) **Europäisches Patentamt / European Patent Office / Office européen des brevets**

(11) **EP 4 678 813 A1**

(12) **EUROPEAN PATENT APPLICATION**
published in accordance with Art. 153(4) EPC

(43) Date of publication:
**14.01.2026   Bulletin 2026/03**

(21) Application number: **24766785.0**

(22) Date of filing: **13.02.2024**

(51) International Patent Classification (IPC):
**D21B 1/06** (2006.01)    **A61F 13/53** (2006.01)
**D21H 11/04** (2006.01)    **D21H 15/02** (2006.01)

(52) Cooperative Patent Classification (CPC):
**A61F 13/53; D21B 1/06; D21H 11/04; D21H 15/02**

(86) International application number:
**PCT/JP2024/004814**

(87) International publication number:
**WO 2024/185413 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.03.2023   JP 2023033310**
**06.03.2023   JP 2023033311**
**06.03.2023   JP 2023033312**
**06.03.2023   JP 2023033313**

(71) Applicant: **Oji Holdings Corporation**
**Chuo-ku**
**Tokyo 104-0061 (JP)**

(72) Inventors:
• **NAKAYAMA Kodai**
**Tokyo 104-0061 (JP)**
• **ISOZAKI Tomofumi**
**Tokyo 104-0061 (JP)**
• **WATANABE Kaori**
**Tokyo 104-0061 (JP)**
• **ISHIKAWA Yumi**
**Tokyo 104-0061 (JP)**

(74) Representative: **Godemeyer Blum Lenze**
**Patentanwälte**
**Partnerschaft mbB - werkpatent**
**An den Gärten 7**
**51491 Overath (DE)**

(54) **PULP SHEET FOR FLUFF PULP, FLUFF MAT, AND ABSORBENT ARTICLE**

(57)    It is an object of the present invention to provide a pulp sheet for fluff pulp, capable of forming a fluff pulp (fluff mat) having a sufficiently reduced liquid backflow percentage. The present invention relates to a pulp sheet for fluff pulp, comprising a hardwood pulp, wherein the content of the hardwood pulp is 25% by mass or more, with respect to the total pulp components in the pulp sheet for fluff pulp, and the total content of straight-chain fatty acid containing 24 carbon atoms, straight-chain fatty acid containing 26 carbon atoms and straight-chain fatty acid containing 28 carbon atoms in the pulp sheet for fluff pulp is 300 to 1000 ppm.

**EP 4 678 813 A1**

**Description**

Technical Field

The present invention relates to a pulp sheet for fluff pulp, a fluff mat, and an absorbent article.

Background Art

Fluff pulp (cotton-like pulp) is made by defibrating wood pulp until it becomes a cotton-like state. Such fluff pulp is used as a water-absorbing material in absorbent articles such as disposable diapers and sanitary napkins.
Conventionally, fluff pulp used in absorbent articles is made from softwood pulp with good defibration properties (for example, Patent Document 1). In Patent Document 1, a fluff pulp is obtained by defibrating a raw material pulp (bleached softwood pulp) having a water content percentage of 35% or less through a mechanical treatment.
In addition, Patent Document 2 discloses a pulp sheet for fluff pulp that contains a bleached hardwood pulp and a bleached softwood pulp. In this document, studies are conducted regarding that the content ratio between the bleached hardwood pulp and the bleached softwood pulp is set to be within a predetermined range to obtain a pulp sheet for fluff pulp that can reduce the backflow amount of an absorbed liquid and can suppress skin rashes.

Prior Art Documents

Patent Documents

Patent Document1: Japanese Patent Publication No. 2012-211411 A
Patent Document2: International Publication WO2022/138870

Summary of Invention

Object to be Solved by the Invention

As mentioned above, a pulp sheet for fluff pulp containing a bleached hardwood pulp and a bleached softwood pulp has been known, but in the conventional pulp sheets for fluff pulp, when a fluff pulp (fluff mat) is produced from the pulp sheets for fluff pulp, it cannot be said that the liquid backflow percentage is sufficiently low, and improvement has been required.
Thus, in order to solve such a problem of the prior art technique, the present inventors have proceeded with studies for the purpose of providing a pulp sheet for fluff pulp that can form a fluff pulp (fluff mat) having a sufficiently reduced liquid backflow percentage.

Means for Solving the Object

Examples of specific aspects of the present invention will be shown below.
[1] A pulp sheet for fluff pulp, comprising a hardwood pulp, wherein

the content of the hardwood pulp is 25% by mass or more, with respect to the total pulp components in the pulp sheet for fluff pulp, and
the total content of straight-chain fatty acid containing 24 carbon atoms, straight-chain fatty acid containing 26 carbon atoms and straight-chain fatty acid containing 28 carbon atoms in the pulp sheet for fluff pulp is 300 to 1000 ppm.

[2] The pulp sheet for fluff pulp according to [1], wherein the ISO brightness measured in accordance with JIS P 8148: 2018 is 80% or more.
[3] The pulp sheet for fluff pulp according to [1] or [2], wherein the viscosity measured in accordance with JIS P 8215: 1998 is 7.0 to 15.0 mPa·s.
[4] The pulp sheet for fluff pulp according to any one of [1] to [3], wherein the kappa number of the hardwood pulp is 0.5 to 2.0.

[5] The pulp sheet for fluff pulp according to any one of [1] to [4], wherein the content of the hardwood pulp is 50% by mass or more, with respect to the total pulp components in the pulp sheet for fluff pulp.

[6] The pulp sheet for fluff pulp according to any one of [1] to [5], wherein the kappa number measured in accordance with JIS P 8211: 2011 is smaller than 1.5.

[7] A pulp sheet for fluff pulp, comprising a hardwood pulp, wherein

the content of xylan in the pulp sheet for fluff pulp is 20% by mass or less, and
when a disintegrated pulp obtained by disintegrating the pulp sheet for fluff pulp in accordance with JIS P 8220-1: 2012 is measured in terms of Canadian Canadian Standard Freeness (CSF) measured in accordance with JIS P 8121: 2012-2, the Canadian Standard Freeness (CSF) is 400 to 640 mL.

[8] The pulp sheet for fluff pulp according to [7], wherein
the ISO brightness measured in accordance with JIS P 8148: 2018 is 82% or more.

[9] The pulp sheet for fluff pulp according to [7] or [8], wherein

when the ISO brightness is measured in accordance with JIS P 8154-1: 2008 before and after heating the pulp sheet for fluff pulp at 105°C for 72 hours, and the ISO brightness reduction percentage is calculated according to the following equation, the brightness change amount is 5.0% or less.

$$\text{Amount (\%) of ISO brightness changed} = B_0\ (\%) - B_1\ (\%)$$

(wherein $B_0$ represents the ISO brightness of the pulp sheet for fluff pulp before the heat treatment, and $B_1$ represents the ISO brightness of the pulp sheet for fluff pulp after the heat treatment.)

[10] The pulp sheet for fluff pulp according to any one of [7] to [9], wherein
the kappa number of the hardwood pulp is 0.3 to 2.0.

[11] The pulp sheet for fluff pulp according to any one of [7] to [10], wherein the content of the hardwood pulp is 25% by mass or more, with respect to the total pulp components in the pulp sheet for fluff pulp.

[12] The pulp sheet for fluff pulp according to any one of [7] to [11], wherein
the kappa number measured in accordance with JIS P 8211: 2011 is smaller than 1.7.

[13] The pulp sheet for fluff pulp according to any one of [7] to [12], wherein the Permanganate number measured in accordance with JIS P 8206: 1982 is smaller than 1.6.

[14] A pulp sheet for fluff pulp, comprising a hardwood pulp, wherein
when the length-weighted fiber length of pulp fibers comprised in the pulp sheet for fluff pulp is measured in accordance with JIS P 8226-2: 2011, the percentage of the number of the fibers weighted by length in the range of 0 to 0.1 mm in the length-weighted fiber length distribution is 10% or less

[15] The pulp sheet for fluff pulp according to [14], wherein
the ISO brightness measured in accordance with JIS P 8148: 2018 is 82% or more.

[16] The pulp sheet for fluff pulp according to [14] or [15], wherein
the kappa number of the hardwood pulp is 0.5 to 2.0.

[17] The pulp sheet for fluff pulp according to any one of [14] to [16], wherein
the kappa number measured in accordance with JIS P 8211: 2011 is smaller than 1.7.

[18] The pulp sheet for fluff pulp according to any one of [14] to [17], wherein
the content of the hardwood pulp is 25% by mass or more, with respect to the total pulp components in the pulp sheet for fluff pulp.

[19] A pulp sheet for fluff pulp, comprising a hardwood pulp, wherein
the fiber coarseness measured by disintegrating the pulp sheet for fluff pulp in accordance with JIS P 8220-1: 2012 is 0.030 to 0.120 mg/m.

[20] The pulp sheet for fluff pulp according to [19], wherein
the length-weighted average fiber length of pulp fibers comprised in the pulp sheet for fluff pulp, which is measured in accordance with JIS P 8226-2: 2011, is 0.65 mm or more and less than 1.50 mm.

[21] The pulp sheet for fluff pulp according to [19] or [20], wherein
the ISO brightness measured in accordance with JIS P 8148: 2018 is 82% or more.

[22] The pulp sheet for fluff pulp according to any one of [19] to [21], wherein
the kappa number of the hardwood pulp is 0.5 to 2.0.

[23] The pulp sheet for fluff pulp according to any one of [19] to [22], wherein
the content of the hardwood pulp is 25% by mass or more, with respect to the total pulp components in the pulp sheet for fluff pulp.

[24] The pulp sheet for fluff pulp according to any one of [19] to [23], wherein the kappa number measured in accordance with JIS P 8211: 2011 is smaller than 1.7.

[25] A fluff mat formed from the pulp sheet for fluff pulp according to any one of [1] to [24].

[26] The fluff mat according to [25], which has a density of 0.058 g/cm$^3$ or more.

[27] An absorbent article, comprising the fluff mat according to [25] or [26].

Advantageous Effects of Invention

According to the present invention, a fluff mat with a reduced backflow percentage can be obtained.

Brief Description of Drawings

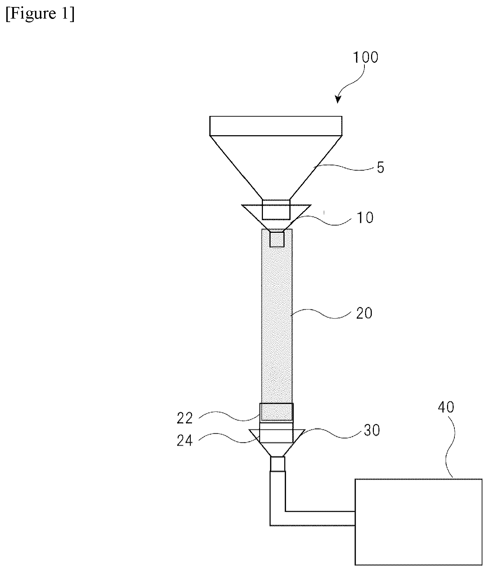

[Figure 1] Figure 1 is a schematic view explaining the configuration of a fluff mat-producing device used in production of fluff mats.

Embodiments of Carrying out the Invention

The present invention will be described in detail below. The constitutive elements of the present invention given below may be described based on some typical embodiments or specific examples. However, the present invention should not be limited to such embodiments. In the present description, the numerical range expressed with the wording "a number to another number" means the range that falls between the former number indicating the lower limit value of the range and the latter number indicating the upper limit value thereof.

(Pulp sheet for fluff pulp)

< First embodiment >

A first embodiment relates to a pulp sheet for fluff pulp, comprising a hardwood pulp, wherein the content of the hardwood pulp is 25% by mass or more, with respect to the total pulp components in the pulp sheet for fluff pulp, and the total content of straight-chain fatty acid containing 24 carbon atoms, straight-chain fatty acid containing 26 carbon atoms and straight-chain fatty acid containing 28 carbon atoms in the pulp sheet for fluff pulp is 300 to 1000 ppm. It is to be noted that the "fluff pulp" in the present description means a defibrated pulp, which is a pulp that has been fiberized or has been cut finely. Also, the "pulp sheet for fluff pulp" is a pulp sheet before defibration, which is used to produce a fluff pulp. In other words, the "fluff pulp" is obtained by defibrating the "pulp sheet for fluff pulp," and the "fluff pulp" that has been molded into a sheet is called a "fluff mat." In the present description, the "pulp sheet for fluff pulp" is obtained, for example, by papermaking a slurry containing a predetermined raw material pulp.

In the first embodiment, the total content of straight-chain fatty acid containing 24 carbon atoms, straight-chain fatty acid containing 26 carbon atoms and straight-chain fatty acid containing 28 carbon atoms in the pulp sheet for fluff pulp is preferably 300 ppm or more, more preferably 320 ppm or more, and further preferably 350 ppm or more. On the other hand, the total content of straight-chain fatty acid containing 24 carbon atoms, straight-chain fatty acid containing 26 carbon atoms and straight-chain fatty acid containing 28 carbon atoms in the pulp sheet for fluff pulp in the pulp sheet for fluff pulp is preferably 1000 ppm or less, more preferably 990 ppm or less, and further preferably 980 ppm or less.

The total content of straight-chain fatty acid containing 24 carbon atoms, straight-chain fatty acid containing 26 carbon atoms and straight-chain fatty acid containing 28 carbon atoms in the pulp sheet for fluff pulp is calculated according to the following method. First, the pulp sheet for fluff pulp is dried, about 200 mg of the dried pulp is precisely weighed, and 0.1 ml of concentrated hydrochloric acid (12 N) and 2 ml of chloroform are added thereto, followed by performing an ultrasonic treatment for 10 minutes. The obtained extract is filtrated though a membrane filter (pore diameter: 0.2 $\mu$m), and an analysis is carried out according to high performance liquid chromatography using the filtrate. As an eluent, here is used methanol : 0.1% by mass of trifluoroacetic acid-containing acetone = 50 : 50, and the flow rate of the high performance liquid chromatography is set to be 1 ml/min. As a column to be used, for example, X Bridge C18, 250 mm x 4.6 mm I.D, manufactured by Waters, can be used. The temperature during the analysis is 30°C, and 2 $\mu$l of the filtrate is injected. As a detector, a charged particle detector is used to detect straight-chain fatty acids (higher fatty acids: $C_{23}H_{47}COOH$, $C_{25}H_{51}COOH$, and $C_{27}H_{55}COOH$), and the concentration of each fatty acid in the sample is obtained using the following calculation formula, and the total concentration is then calculated. The peak area value of each component is the area surrounded by the baseline and each peak, but for partially overlapping peaks, the peak area can be separated and calculated using the perpendicular line method of JIS K 0124: 2011.

In the first embodiment, by appropriately adjusting, for example, raw materials for the pulp, production conditions for the pulp, and papermaking conditions for the pulp sheet for fluff pulp, the total content of straight-chain fatty acid containing 24 carbon atoms, straight-chain fatty acid containing 26 carbon atoms and straight-chain fatty acid containing 28 carbon atoms in the pulp sheet for fluff pulp can be controlled within the above-described range.

In the first embodiment, by setting the total content of straight-chain fatty acid containing 24 carbon atoms, straight-chain fatty acid containing 26 carbon atoms and straight-chain fatty acid containing 28 carbon atoms in the pulp sheet for fluff pulp to be within a predetermined range, generation of dirts is suppressed, and a pulp sheet for fluff pulp with good appearance can be obtained. Herein, the appearance of the pulp sheet for fluff pulp is determined to be good when the number of dirts (impurities) observed per 100 g of the pulp sheet for fluff pulp is small. Specifically, the number of dirts (impurities) observed per 100 g of the pulp sheet for fluff pulp is preferably 19 or less, more preferably 15 or less, further preferably 10 or less, and particularly preferably 8 or less. It is to be noted that the number of dirts (impurities) observed per 100 g of the pulp sheet for fluff pulp is most preferably 0. The straight-chain fatty acids contained in the pulp are derived from the pulp raw materials, and the straight-chain fatty acids gradually aggregate during the pulp production step to swell to the size of dirts. Thus, it is considered that the number of dirts decreases as the total content of the above-described straight-chain fatty acids is lower.

Moreover, in the first embodiment, by setting the content of the hardwood pulp with respect to the total pulp components in the pulp sheet for fluff pulp to be a predetermined amount or more, and further, by setting the total content of straight-chain fatty acid containing 24 carbon atoms, straight-chain fatty acid containing 26 carbon atoms and straight-chain fatty acid containing 28 carbon atoms in the pulp sheet for fluff pulp to be within a predetermined range, the liquid backflow percentage can be reduced when a fluff mat is formed from the pulp sheet for fluff pulp. By setting the total content of the above-described straight-chain fatty acids to be the above-described lower limit value or more, it is considered that damage to the pulp fibers can be reduced and the liquid-retaining property can be increased.

The liquid backflow percentage in a fluff mat can be calculated by the following formula. Herein, the liquid backflow percentage in a fluff mat can be calculated by absorbing 20 g of artificial urine into a fluff mat, placing 10 sheets of filter papers (manufactured by ADVANTEC, No. 1640) on the fluff mat while applying pressure to the filter papers under the conditions described in the Examples, and measuring the weight of a liquid absorbed by the filter papers.

[Expression 1]

$$\frac{(\text{Weight [g] of 10 filter papers after liquid absorption}) - (\text{Weight [g] of 10 filter papers before liquid absorption})}{20 \text{ [g]}} \times 100 \text{ [\%]}$$

Specifically, the liquid backflow percentage in the fluff mat is preferably less than 81%, more preferably 80% or less, further preferably 79% or less, and particularly preferably 75% or less.

Furthermore, since the pulp sheet for fluff pulp of the first embodiment has the above-described configuration, its defibration properties are favorable when the pulp sheet is fluffed. In the present description, the defibration properties can be evaluated by the defibration time (sec) required for the undefibrated material to disappear. Specifically, the shorter the defibration time, the better the defibration properties that can be determined, and the better the defibration properties, the higher the production efficiency of the fluff mat that can be obtained. Further, the better the defibration properties, the more likely it is that a liquid fluff mat with a reduced backflow percentage can be obtained. Further, the better the defibration properties, the less the amount of an undefibrated material in the fluff mat, so not only does the appearance of the fluff mat improve, but the ability of the fluff mat to maintain its shape is improved, and when the fluff mat is used as an absorbent layer for sanitary materials such as disposable diapers, the possibility of the shape of the absorbent layer collapsing due to the movement during use and the resulting deterioration of the absorbent performance of the disposable diapers can be reduced.

The ISO brightness of the pulp sheet for fluff pulp of the first embodiment, which is measured in accordance with JIS P 8148: 2018, preferably 80% or more, more preferably 81% or more, further preferably 82% or more, and particularly preferably 83% or more. On the other hand, the ISO brightness of the pulp sheet for fluff pulp is preferably 93% or less. By setting the ISO brightness of the pulp sheet for fluff pulp to be within the above-described range, a pulp sheet for fluff pulp with more excellent appearance can be obtained. In addition, by setting the ISO brightness of the pulp sheet for fluff pulp to be within the above-described range, a fluff mat with high brightness can be obtained.

The viscosity of the pulp sheet for fluff pulp of the first embodiment, which is measured in accordance with JIS P 8215: 1998, is preferably 7.0 mPa·s or more, more preferably 7.5 mPa·s or more, further preferably 8.0 mPa·s or more, still further preferably 8.2 mPa·s or more, and particularly preferably 8.4 mPa·s or more. On the other hand, the viscosity of the pulp sheet for fluff pulp measured in accordance with JIS P 8215: 1998 is preferably 15.0 mPa·s or less, more preferably 14.7 mPa·s or less, and further preferably 14.5 mPa·s or less. Besides, upon the measurement of the viscosity of the pulp sheet for fluff pulp, the viscosity is measured after a viscosity measurement liquid is obtained under the conditions described in JIS P 8215: 1998. By setting the viscosity measured for the pulp sheet for fluff pulp to be within the above-described range, a pulp sheet for fluff pulp having more excellent defibration properties can be obtained.

The kappa number of the pulp sheet for fluff pulp of the first embodiment, which is measured in accordance with JIS P 8211: 2011, is preferably smaller than 1.5, more preferably 1.4 or less, and further preferably 1.3 or less. The lower limit value of the kappa number of the pulp sheet for fluff pulp is not particularly limited, and it is preferably, for example, 0.1 or more. By setting the kappa number of the pulp sheet for fluff pulp to be within the above-described range, a pulp sheet for fluff pulp having high brightness and more excellent defibration properties can be obtained.

The basis weight of the pulp sheet for fluff pulp of the first embodiment is not particularly limited, and the pulp sheet may be produced with a basis weight that enables production by a papermaking device used for papermaking. For example, the basis weight of the pulp sheet for fluff pulp is preferably 100 $g/m^2$ or more, more preferably 150 $g/m^2$ or more, and further preferably 200 $g/m^2$ or more. On the other hand, the basis weight of the pulp sheet for fluff pulp is preferably 1500 $g/m^2$ or less, more preferably 1400 $g/m^2$ or less, and further preferably 1300 $g/m^2$ or less. The basis weight of the pulp sheet for fluff pulp is measured in accordance with JIS P 8124: 2011.

The density of the pulp sheet for fluff pulp of the first embodiment is not particularly limited, and it is preferably 0.3 $g/cm^3$ or more, more preferably 0.4 $g/cm^3$ or more, and further preferably 0.5 $g/cm^3$ or more. On the other hand, the density of the pulp sheet for fluff pulp is preferably 0.95 $g/cm^3$ or less, more preferably 0.90 $g/cm^3$ or less, and further preferably 0.85 $g/cm^3$ or less. The density of the pulp sheet for fluff pulp is measured in accordance with JIS P 8118: 2014.

The burst index of the pulp sheet for fluff pulp of the first embodiment, which is measured in accordance with JIS P 8131: 2009, is preferably 0.5 $kPa \cdot m^2/g$ or more, more preferably 0.7 $kPa \cdot m^2/g$ or more, and further preferably 0.9 $kPa \cdot m^2/g$ or more. On the other hand, the burst index of the pulp sheet for fluff pulp is preferably 5.0 $kPa \cdot m^2/g$ or less, more preferably 4.5 $kPa \cdot m^2/g$ or less, and further preferably 4.0 $kPa \cdot m^2/g$ or less.

< Second embodiment >

A second embodiment relates to a pulp sheet for fluff pulp, comprising a hardwood pulp, wherein the content of xylan in the pulp sheet for fluff pulp is 20% by mass or less, and when a disintegrated pulp obtained by disintegrating the pulp sheet for fluff pulp in accordance with JIS P 8220-1: 2012 is measured in terms of Canadian Standard Freeness (CSF) measured in accordance with JIS P 8121: 2012-2, Canadian Standard Freeness (CSF) is 400 to 640 mL.

In the second embodiment, the content of xylan in the pulp sheet for fluff pulp is preferably 20% by mass or less, more preferably 18% by mass or less, and further preferably 17% by mass or less. The lower limit value of the content of xylan in the pulp sheet for fluff pulp is not particularly limited, and it may be 0% by mass.

The content of xylan in the pulp sheet for fluff pulp is calculated using the following equations, based on the results of the quantitative analysis of xylose contained in the pulp sheet for fluff pulp. It is to be noted that the xylose can be quantified using ion chromatography, after the acid hydrolysis of the pulp sheet for fluff pulp has been carried out using sulfuric acid according to an NREL/TP-510-42618 method.

Amount of xylan (g) = amount of xylose measured ($\mu$M) $\times$ 132 (molecular weight of xylose unit)/overdecomposition percentage of sugar by acid (%) Amount of xylan (%) = amount of xylan (g)/mass of pulp sheet for fluff pulp subjected to ion chromatography (g)

In the second embodiment, when a disintegrated pulp obtained by disintegrating the pulp sheet for fluff pulp in accordance with JIS P 8220-1: 2012 is measured in terms of Canadian Standard Freeness (CSF) measured in accordance with JIS P 8121: 2012-2, Canadian Standard Freeness (CSF) is preferably 400 mL or more, more preferably 420 mL or more, and further preferably 450 mL or more. On the other hand, the above-described Canadian Standard Freeness (CSF) is preferably 640 mL or less, more preferably 620 mL or less, and further preferably 600 mL or less.

In the second embodiment, by appropriately adjusting, for example, raw materials for the pulp, production conditions for the pulp, papermaking conditions for the pulp sheet for fluff pulp, and beating conditions, it becomes possible to control the content of xylan in the pulp sheet for fluff pulp to be within the above-described range, and further, the above-described Canadian Standard Freeness (CSF) can be controlled to be within a predetermined range.

In the second embodiment, by setting the content of xylan in the pulp sheet for fluff pulp to be within a predetermined range, and further, by setting the Canadian Standard Freeness (CSF) of the disintegrated pulp to be within a predetermined range, a pulp sheet for fluff pulp having a good defibration properties can be obtained. In the present description, the defibration properties can be evaluated by the defibration time (sec) required for the undefibrated material to disappear. Specifically, the shorter the defibration time, the better the defibration properties that can be determined, and the better the defibration properties, the higher the production efficiency of the fluff mat that can be obtained. Further, the better the defibration properties, the more likely it is that a liquid fluff mat with a reduced backflow percentage can be obtained. Further, the better the defibration properties, the less the amount of an undefibrated material in the fluff mat, so not only does the appearance of the fluff mat improve, but the ability of the fluff mat to maintain its shape is improved, and when the fluff mat is used as an absorbent layer for sanitary materials such as disposable diapers, the possibility of the shape of the absorbent layer collapsing due to the movement during use and the resulting deterioration of the absorbent performance of the disposable diapers can be reduced.

In addition, in the second embodiment, by setting the content of xylan in the pulp sheet for fluff pulp to be within a predetermined range, and further, by setting the Canadian Standard Freeness (CSF) of the disintegrated pulp to be within a predetermined range, the liquid backflow percentage can be reduced when a fluff mat is formed from the pulp sheet for fluff pulp.

In the second embodiment, by setting the content of xylan to be within a predetermined range, it is considered that the chemical bond strength between pulp fibers can be reduced, and that the defibration properties can be improved. In addition, by setting the Canadian Standard Freeness (CSF) to be within a predetermined range, it is considered that the degree of entanglement and hydrogen bonding between pulp fibers in the pulp sheet for fluff pulp can be weakened, thereby improving the defibration properties and improving the water retentivity of the pulp fibers, so that the backflow percentage of the fluff mat can be reduced.

The liquid backflow percentage in a fluff mat can be calculated by the following formula. Herein, the liquid backflow percentage in a fluff mat can be calculated by absorbing 20 g of artificial urine into a fluff mat, placing 10 sheets of filter papers (manufactured by ADVANTEC, No. 1640) on the fluff mat while applying pressure to the filter papers under the conditions described in the Examples, and measuring the weight of a liquid absorbed by the filter papers.

[Expression 2]

$$\frac{(\text{Weight [g] of 10 filter papers after liquid absorption}) - (\text{Weight [g] of 10 filter papers before liquid absorption})}{20 \ [g]} \times 100 \ [\%]$$

Specifically, the liquid backflow percentage in the fluff mat is preferably 85% or less, more preferably 82% or less, further preferably 80% or less, and particularly preferably 79% or less.

Furthermore, in the second embodiment, by setting the content of xylan in the pulp sheet for fluff pulp to be within a predetermined range, and further, by setting the Canadian Standard Freeness (CSF) of the disintegrated pulp to be within a predetermined range, a pulp sheet for fluff pulp with suppressed color fading and excellent appearance can be obtained. In the present description, suppression of color fading can be evaluated by a change in the ISO brightness before and after a heat treatment. Specifically, the pulp sheet for fluff pulp is subjected to a heat treatment at 105°C for 72 hours in accordance with JIS P 8154-1: 2008, and the ISO brightness before and after the heat treatment is measured in accordance with JIS P 8148: 2018. Then, the change amount in the ISO brightness is calculated using the following equation:

$$\text{Change amount of ISO brightness (\%)} = B_0 \ (\%) - B_1 \ (\%)$$

In the above equation, $B_0$ indicates the ISO brightness of the pulp sheet for fluff pulp before the heat treatment, and $B_1$ indicates the ISO brightness of the pulp sheet for fluff pulp after the heat treatment.

In the second embodiment, the change amount in the ISO brightness is preferably 5.0% or less, more preferably 4.8% or less, further preferably 4.6% or less, still further preferably 4.4% or less, and particularly preferably 4.2% or less. When the change amount in the ISO brightness is within the above-described range, the pulp sheet for fluff pulp can be evaluated to have suppressed color fading and excellent appearance.

In the pulp sheet for fluff pulp of the second embodiment, the ISO brightness measured in accordance with JIS P 8148: 2018 (ISO brightness before heating) is preferably 80% or more, more preferably 81% or more, further preferably 82% or more, and particularly preferably 83% or more. On the other hand, the ISO brightness of the pulp sheet for fluff pulp is preferably 93% or less. By setting the ISO brightness of the pulp sheet for fluff pulp to be within the above-described range, a pulp sheet for fluff pulp having more excellent appearance can be obtained. In addition, by setting the ISO brightness of the pulp sheet for fluff pulp to be within the above-described range, a fluff mat having high brightness can be obtained.

In the pulp sheet for fluff pulp of the second embodiment, the kappa number measured in accordance with JIS P 8211: 2011 is preferably smaller than 1.7, more preferably smaller than 1.6, further preferably 1.5 or less, and particularly preferably 1.4 or less. The lower limit value of the kappa number of the pulp sheet for fluff pulp is not particularly limited, and it is preferably, for example, 0.1 or more. By setting the kappa number of the pulp sheet for fluff pulp to be within the above-described range, a pulp sheet for fluff pulp having high brightness and more excellent defibration properties can be obtained.

In the pulp sheet for fluff pulp of the second embodiment, the Permanganate number measured in accordance with JIS P 8206: 1982 is preferably smaller than 1.6, more preferably 1.5 or less, and further preferably 1.4 or less. The lower limit value of the Permanganate number of the pulp sheet for fluff pulp is not particularly limited, and it is preferably, for example, 0.1 or more. By setting the Permanganate number of the pulp sheet for fluff pulp to be within the above-described range, a pulp sheet for fluff pulp having high brightness and more excellent defibration properties can be obtained.

The preferred numerical value ranges of the basis weight, density, and also, burst index measured in accordance with JIS P 8131: 2009, of the pulp sheet for fluff pulp of the second embodiment are the same as those of the basis weight, density, and burst index of the pulp sheet for fluff pulp of the first embodiment.

< Third embodiment >

A third embodiment relates to a pulp sheet for fluff pulp, comprising a hardwood pulp, wherein when the length-weighted fiber length of the pulp fibers comprised in the pulp sheet for fluff pulp is measured in accordance with JIS P 8226-2: 2011, the percentage of the number of fibers having a length-weighted fiber length of 0.1 mm or less to the total number of pulp fibers comprised in the pulp sheet for fluff pulp is 10% or less.

In the present description, when the length-weighted fiber length of the pulp fibers comprised in the pulp sheet for fluff pulp is measured in accordance with JIS P 8226-2: 2011, the percentage of the number of fibers with a fiber length of 0.1 mm or less in the length-weighted fiber length distribution is also referred to as the amount of fines (%). The amount of fines (%) in the pulp sheet for fluff pulp is preferably 10% or less, more preferably 9.5% or less, and further preferably 9.0% or less. The lower limit value of the amount of fines (%) in the pulp sheet for fluff pulp is not particularly limited, and it may be 0%.

Herein, the length-weighted fiber length of the pulp fibers comprised in the pulp sheet for fluff pulp is measured in accordance with JIS P 8226-2: 2011. Since the length-weighted fiber length of pulp fibers is the disintegrated fiber length of pulp fibers, the length-weighted fiber length is measured after disintegration in accordance with JIS P 8226-2: 2011. The percentage of the number of the fibers weighted by length in the range of 0 to 0.1 mm in the distribution of the obtained length-weighted fiber length is calculated as the amount of fines.

In the third embodiment, by appropriately adjusting, for example, the conditions for production of the pulp sheet for fluff pulp, the type of a pulp used, and the presence or absence of a classification step for the pulp fibers and the classification conditions, the amount of fines comprised in the pulp sheet for fluff pulp can be controlled to be within the above-described range.

In the third embodiment, by setting the amount of fines comprised in the pulp sheet for fluff pulp to be within a predetermined range, a sheet for fluff pulp having good defibration properties can be obtained. In the present description, the defibration properties can be evaluated by the defibration time (sec) required for the undefibrated material to disappear. Specifically, the shorter the defibration time, the better the defibration properties that can be determined, and the better the defibration properties, the higher the production efficiency of the fluff mat that can be obtained. Further, the better the defibration properties, the more likely it is that a liquid fluff mat with a reduced backflow percentage can be obtained. Further, the better the defibration properties, the less the amount of an undefibrated material in the fluff mat, so not only does the appearance of the fluff mat improve, but the ability of the fluff mat to maintain its shape is improved, and when the fluff mat is used as an absorbent layer for sanitary materials such as disposable diapers, the possibility of the shape of the absorbent layer collapsing due to the movement during use and the resulting deterioration of the absorbent performance of the disposable diapers can be reduced.

In addition, in the third embodiment, by setting the amount of fines comprised in the pulp sheet for fluff pulp to be within a predetermined range, the liquid backflow percentage can be reduced when a fluff mat is formed from the pulp sheet for fluff pulp.

The liquid backflow percentage in a fluff mat can be calculated by the following formula. Herein, the liquid backflow percentage in a fluff mat can be calculated by absorbing 20 g of artificial urine into a fluff mat, placing 10 sheets of filter papers (manufactured by ADVANTEC, No. 1640) on the fluff mat while applying pressure to the filter papers under the conditions described in the Examples, and measuring the weight of a liquid absorbed by the filter papers.

[Expression 3]

$$\frac{(\text{Weight [g] of 10 filter papers after liquid absorption}) - (\text{Weight [g] of 10 filter papers before liquid absorption})}{20\ [\text{g}]} \times 100\ [\%]$$

Specifically, the liquid backflow percentage in the fluff mat is preferably 85% or less, more preferably 82% or less, further preferably 80% or less, and particularly preferably 79% or less.

Moreover, in the third embodiment, even in a diaper comprising the fluff mat, the backflow percentage can be reduced. The liquid backflow percentage in such a diaper can be calculated by the following formula. Specifically, the liquid backflow percentage in a fluff mat (pseudo diaper) formed by uniformly sprinkling highly absorbent polymers can be calculated by absorbing 20 g of artificial urine into the fluff mat, placing 10 sheets of filter papers (manufactured by ADVANTEC, No. 1640) on the fluff mat while applying pressure to the filter papers under the conditions described in the Examples, and measuring the weight of a liquid absorbed by the filter papers.

[Expression 4]

$$\frac{\text{(Weight [g] of 10 filter papers after liquid absorption)} - \text{(Weight [g] of 10 filter papers before liquid absorption)}}{20\,[g]} \times 100\,[\%]$$

Specifically, the liquid backflow percentage in the diaper is preferably 20% or less, more preferably 15% or less, and further preferably 10% or less.

As the amount of fines comprised in the pulp sheet for fluff pulp is increased, the water retentivity is improved. Thus, when a pressure is applied after the fluff mat has been allowed to absorb a liquid, the liquid backflow percentage is decreased. On the other hand, as the amount of fines comprised in the pulp sheet for fluff pulp is decreased, the size of the voids in the pulp sheet for fluff pulp is increased, and thus, the defibration properties is improved. In this way, in the third embodiment, by setting the amount of fines to be within a predetermined range, it becomes possible to achieve both the backflow percentage and the defibration properties.

In the pulp sheet for fluff pulp of the third embodiment, the ISO brightness measured in accordance with JIS P 8148: 2018 is preferably 80% or more, more preferably 81% or more, further preferably 82% or more, and particularly preferably 83% or more. On the other hand, the ISO brightness of the pulp sheet for fluff pulp is preferably 93% or less. By setting the ISO brightness of the pulp sheet for fluff pulp to be within the above-described range, a pulp sheet for fluff pulp having more excellent appearance can be obtained. In addition, by setting the ISO brightness of the pulp sheet for fluff pulp to be within the above-described range, a fluff mat having high brightness can be obtained.

In the pulp sheet for fluff pulp of the third embodiment, the kappa number measured in accordance with JIS P 8211: 2011 is preferably smaller than 1.7, more preferably smaller than 1.6, further preferably 1.5 or less, still further preferably smaller than 1.5, and particularly preferably 1.4 or less. The lower limit value of the kappa number of the pulp sheet for fluff pulp is not particularly limited, and it is preferably, for example, 0.1 or more. By setting the kappa number of the pulp sheet for fluff pulp to be within the above-described range, pulp a sheet for fluff pulp having high brightness and more excellent defibration properties can be obtained.

The preferred numerical value ranges of the basis weight, density, and also, burst index measured in accordance with JIS P 8131: 2009, of the pulp sheet for fluff pulp of the third embodiment are the same as those of the basis weight, density, and burst index of the pulp sheet for fluff pulp of the first embodiment.

< Fourth embodiment >

A fourth embodiment relates to a pulp sheet for fluff pulp, comprising a hardwood pulp, wherein the fiber coarseness measured by disintegrating the pulp sheet for fluff pulp in accordance with JIS P 8220-1: 2012 is 0.03 to 0.12 mg/m.

In the present embodiment, the fiber coarseness measured by disintegrating the pulp sheet for fluff pulp in accordance with JIS P 8220-1: 2012 is preferably 0.030 mg/m or more, more preferably 0.035 mg/m or more, and further preferably 0.040 mg/m or more. On the other hand, the fiber coarseness measured by disintegrating the pulp sheet for fluff pulp is 0.120 mg/m or less, and more preferably 0.110 mg/m or less.

In the present embodiment, by appropriately adjusting, for example, raw materials for the pulp, production conditions for the pulp, and papermaking conditions for the pulp sheet for fluff pulp, the fiber coarseness measured by disintegrating the pulp sheet for fluff pulp can be controlled within the above-described range.

In the present embodiment, by setting the fiber coarseness measured by disintegrating the pulp sheet for fluff pulp to be within a predetermined range, a fluff mat having a high density can be obtained, and as a result, a fluff mat having a thin thickness can be formed. Specifically, the density of the obtained fluff mat is preferably 0.058 g/cm$^3$ or more, and more preferably 0.060 g/cm$^3$ or more. On the other hand, the density of the obtained fluff mat is preferably 0.150 g/cm$^3$ or less.

Moreover, in the present embodiment, by setting the fiber coarseness measured by disintegrating the pulp sheet for fluff pulp to be within a predetermined range, when a fluff mat is formed from the pulp sheet for fluff pulp, the liquid backflow percentage can be reduced.

The liquid backflow percentage in a fluff mat can be calculated by the following formula. Herein, the liquid backflow percentage in a fluff mat can be calculated by absorbing 20 g of artificial urine into a fluff mat, placing 10 sheets of filter papers (manufactured by ADVANTEC, No. 1640) on the fluff mat while applying pressure to the filter papers under the conditions described in the Examples, and measuring the weight of a liquid absorbed by the filter papers.

## [Expression 5]

$$\frac{(\text{Weight [g] of 10 filter papers after liquid absorption}) - (\text{Weight [g] of 10 filter papers before liquid absorption})}{20\ [\text{g}]} \times 100\ [\%]$$

Specifically, the liquid backflow percentage in the fluff mat is preferably 85% or less, more preferably 82% or less, further preferably 80% or less, and particularly preferably 79% or less.

Fiber coarseness is an index related to the stiffness of pulp fibers. By reducing the fiber coarseness of the pulp fibers comprised in the pulp sheet for fluff pulp, the size of the voids in the fluff mat becomes smaller, and thus, the density of the fluff mat increases. In addition, since capillary action becomes easier to work, it is considered that the ability to retain liquid in the voids in the fluff mat is improved, and thus that the liquid backflow percentage can be reduced.

The length-weighted average fiber length of the pulp fibers comprised in the pulp sheet for fluff pulp of the present embodiment, which is measured in accordance with JIS P 8226-2: 2011, is preferably 0.65 mm or more, and more preferably 0.70 mm or more. On the other hand, the length-weighted average fiber length of the pulp fibers comprised in the pulp sheet for fluff pulp, which is measured in accordance with JIS P 8226-2: 2011, is preferably less than 1.50 mm, more preferably 1.45 mm or less, and further preferably 1.40 mm or less.

Herein, the length-weighted average fiber length of the pulp fibers comprised in the pulp sheet for fluff pulp is measured in accordance with JIS P 8226-2: 2011. Since the length-weighted fiber length of pulp fibers is the disintegrated fiber length of the pulp fibers, the length-weighted average fiber length is measured after disintegration in accordance with JIS P 8226-2: 2011.

In the present embodiment, by setting the length-weighted average fiber length of the pulp fibers comprised in the pulp sheet for fluff pulp to be within a predetermined range, a fluff mat having a high density can be easily obtained, and as a result, a fluff mat having a thin thickness can be easily formed. In addition, in the present embodiment, by setting the length-weighted average fiber length of the pulp fibers comprised in the pulp sheet for fluff pulp to be within a predetermined range, when a fluff mat is formed from the pulp sheet for fluff pulp, the liquid backflow percentage can be more effectively reduced.

In the pulp sheet for fluff pulp of the present embodiment, the ISO brightness measured in accordance with JIS P 8148: 2018 is preferably 80% or more, more preferably 81% or more, further preferably 82% or more, and particularly preferably 83% or more. On the other hand, the ISO brightness of the pulp sheet for fluff pulp is preferably 93% or less. By setting the ISO brightness of the pulp sheet for fluff pulp to be within the above-described range, a pulp sheet for fluff pulp having more excellent appearance can be obtained. In addition, by setting the ISO brightness of the pulp sheet for fluff pulp to be within the above-described range, a fluff mat having high brightness can be obtained.

In the pulp sheet for fluff pulp of the present embodiment, the kappa number measured in accordance with JIS P 8211: 2011 is preferably smaller than 1.7, more preferably smaller than 1.6, further preferably 1.5 or less, and particularly preferably 1.4 or less. The lower limit value of the kappa number of the pulp sheet for fluff pulp is not particularly limited, and it is preferably, for example, 0.1 or more. By setting the kappa number of the pulp sheet for fluff pulp to be within the above-described range, pulp a sheet for fluff pulp having high brightness and more excellent defibration properties can be obtained.

The preferred numerical value ranges of the basis weight, density, and also, burst index measured in accordance with JIS P 8131: 2009, of the pulp sheet for fluff pulp of the fourth embodiment are the same as those of the basis weight, density, and burst index of the pulp sheet for fluff pulp of the first embodiment.

(Raw material pulp)

The pulp sheet for fluff pulp of the present embodiment (hereinafter, the first embodiment to the fourth embodiment are collectively referred to as the present embodiment) comprises a hardwood pulp. The hardwood pulp is preferably a bleached hardwood pulp. The raw material for the bleached hardwood pulp preferably comprises at least one selected from the group consisting of genus Acacia, genus Eucalyptus, genus Quercus, genus Fagus, and genus Castanopsis, and more preferably comprises at least one selected from the group consisting of genus Acacia and genus Eucalyptus. Moreover, in the present embodiment, as raw materials for the bleached hardwood pulp, both genus Acacia and genus Eucalyptus are preferably used in combination. In particular, by allowing the pulp sheet for fluff pulp to comprise a bleached hardwood pulp produced from genus Acacia, when the pulp sheet for fluff pulp has been fluffed, the liquid absorbency of the fluff mat can be more effectively increased, and the liquid backflow percentage can be more effectively reduced. Besides, the above-described raw materials can be used, as appropriate, for the raw materials of the bleached hardwood pulp, and in particular, in the second embodiment, it is preferable to control the mixed amount of the raw materials, depending on the content of xylan contained in each raw material.

The content of the hardwood pulp is preferably 25% by mass or more, more preferably 30% by mass or more, further preferably 35% by mass or more, even further preferably 40% by mass or more, still further preferably 45% by mass or more, and particularly preferably 50% by mass or more, with respect to the total pulp components in the pulp sheet for fluff pulp. The upper limit value of the content of the hardwood pulp with respect to the total pulp components in the pulp sheet for fluff pulp is not particularly limited, and it may be, for example, 100% by mass.

The kappa number of the hardwood pulp used as a raw material pulp is preferably 0.3 or more, more preferably 0.35 or more, further preferably 0.4 or more, even further preferably 0.5 or more, still further preferably 0.75 or more, and particularly preferably 1.0 or more. On the other hand, the kappa number of the hardwood pulp is preferably 2.0 or less, more preferably 1.8 or less, further preferably 1.6 or less, and particularly preferably 1.5 or less. In the present embodiment, by setting the kappa number of the hardwood pulp to be within the above-described range, when the pulp sheet for fluff pulp has been fluffed, the liquid backflow percentage of the fluff mat can be more effectively reduced. In addition, by setting the kappa number of the hardwood pulp to be the above-described lower limit value or more, the amount of a bleaching chemical drug used during pulp production can not only be reduced, but also stiff fibers with less damage can be formed. Accordingly, the defibration properties of the pulp sheet for fluff pulp can be improved, and the amount of fines can be reduced.

The Canadian Standard Freeness (CSF) of the hardwood pulp measured in accordance with JIS P 8121-2: 2012 is preferably 370 ml or more, more preferably 400 ml or more, and further preferably 430 ml or more. It is to be noted that this Canadian Standard Freeness (CSF) of the hardwood pulp is a value measured on a hardwood pulp before papermaking.

The pulp sheet for fluff pulp of the present embodiment may comprise other raw material pulps, in addition to the hardwood pulp. Examples of such other raw material pulps may include a softwood pulp, a non-wood pulp, and a deinked pulp. Examples of the hardwood pulp and the softwood pulp may include chemical pulps such as a sulfite pulp (SP), a soda pulp (AP), an unbleached kraft pulp (UKP) and an oxygen bleached kraft pulp (OKP), semichemical pulps such as a semi-chemical pulp (SCP) and a chemiground wood pulp (CGP), and mechanical pulps such as a ground pulp (GP) and a thermomechanical pulp (TMP, BCTMP). In addition, such other raw material may also be a dissolving pulp (DP). Examples of the non-wood pulp may include cotton pulps such as cotton linter and cotton lint, bamboo pulps, and non-wood type pulps such as hemp, wheat straw, and bagasse. An example of the deinked pulp may be a deinked pulp using waste paper as a raw material. Among others, as raw material pulps, a bleached hardwood pulp and a bleached softwood pulp are preferably used in combination. By allowing the pulp sheet for fluff pulp to comprise the bleached hardwood pulp and the bleached softwood pulp, when the pulp sheet for fluff pulp has been fluffed, the density of the fluff mat can be set within an appropriate range, and the liquid backflow percentage of the fluff mat can be more effectively reduced.

That content of the softwood pulp is preferably 75% by mass or less, more preferably 70% by mass or less, further preferably 65% by mass or less, even further preferably 60% by mass or less, still further preferably 55% by mass or less, and particularly preferably 50% by mass or less, with respect to the total pulp components in the pulp sheet for fluff pulp softwood pulp. On the other hand, the lower limit value of the content of the hardwood pulp with respect to the total pulp components in the pulp sheet for fluff pulp is not particularly limited, and it may be, for example, 0% by mass, and it is preferably 5% by mass or more, and more preferably 10% by mass or more.

When a softwood pulp is comprised in the pulp sheet for fluff pulp, the softwood pulp is preferably a bleached softwood pulp. The raw materials for the bleached softwood pulp preferably comprise at least one selected from the group consisting of genus Pinus, genus Pseudotsuga and genus Cryptomeria, and more preferably comprise at least one selected from the group consisting of genus Pinus and genus Pseudotsuga. Moreover, in the present embodiment, genus Pinus and genus Pseudotsuga are preferably used in combination.

The Canadian Standard Freeness (CSF) of the softwood pulp measured in accordance with JIS P 8121-2: 2012 is preferably 600 ml or more, more preferably 640 ml or more, and further preferably 670 ml or more. It is to be noted that this Canadian Standard Freeness (CSF) of the softwood pulp is a value measured on a softwood pulp before papermaking.

The pulp sheet for fluff pulp may comprise other pulps, in addition to the hardwood pulp and the softwood pulp. Such other pulps may include various types of pulps mentioned above. In this case, the content of the other pulps is preferably 10% by mass or less with respect to the total pulp components in the pulp sheet for fluff pulp.

The length-weighted average fiber length of the hardwood pulp used as a raw material pulp is preferably 0.50 mm or more, more preferably 0.60 mm or more, and further preferably 0.65 mm or more. On the other hand, the length-weighted average fiber length of the hardwood pulp is preferably 1.20 mm or less, more preferably 1.10 mm or less, and further preferably 1.0 mm or less.

Moreover, the pulp sheet for fluff pulp further comprises a softwood pulp as a raw material pulp, the length-weighted average fiber length of the softwood pulp is preferably 1.50 mm or more, more preferably 1.75 mm or more, and further preferably 2.00 mm or more. On the other hand, the length-weighted average fiber length of the softwood pulp is preferably 3.50 mm or less, more preferably 3.20 mm or less, and further preferably 3.00 mm or less.

By setting the fiber lengths of various types of pulps comprised in the pulp sheet for fluff pulp to be within the above-described ranges, the thickness and density of the pulp sheet for fluff pulp can be easily controlled to be in appropriate ranges. In addition, by setting the fiber lengths of various types of pulps comprised in the pulp sheet for fluff pulp to be within the above-described ranges, when the pulp sheet for fluff pulp has been fluffed, the liquid backflow percentage of the fluff mat can be more effectively reduced. It is to be noted that the length-weighted average fiber lengths of various types of pulps comprised in the pulp sheet for fluff pulp are measured in accordance with JIS P 8226-2: 2011.

(Optional components)

The pulp sheet for fluff pulp may comprise additives, as necessary. Examples of the additives may include a softener, a filler, a pigment, a sizing agent, a coagulant, an oil-resistant agent, aluminum sulfate, a retention aid, a filtration aid, a dry strength agent, a wet strength agent, a color pigment, a water-resistant agent, an antistatic agent, sodium sulfate, a surfactant, and a pH adjuster (sodium hydroxide, etc.). The pulp sheet for fluff pulp of the present embodiment may comprise a softener. By allowing the pulp sheet for fluff pulp to comprise a softener, defibration properties can be more effectively increased.

(Method for producing pulp sheet for fluff pulp)

The method for producing the pulp sheet for fluff pulp of the present embodiment preferably comprises a cooking step of cooking wood chips including hardwood to produce an unbleached pulp, an oxygen delignification step, a bleaching step, and a sheet formation step. In addition, a dewatering and washing step or a fiber classification step may be appropriately provided between the above-described steps, and a prehydrolysis step may be provided before the cooking step, and a cold caustic extraction step may be provided after the bleaching step. Moreover, a beating step of beating a bleached pulp to a desired freeness may be provided before the sheet formation step.

(Cooking step)

In the cooking step, a hardwood or a softwood is cooked to obtain an unbleached pulp. A known method can be adopted as a cooking method. The cooking conditions applied in the cooking step are not particularly limited. For example, when a kraft cooking method is used, the degree of sulfidation of a cooking liquid is preferably 15% to 45%, the cooking temperature is preferably 130°C to 170°C, the effective alkali addition percentage is preferably 5% to 30% by mass per absolute dry wood mass, and the cooking time is preferably 45 to 500 minutes. The kappa number of an unbleached pulp obtained through the cooking step is preferably 10 to 70.

In the cooking step, it is preferable to perform cooking by split addition of a cooking liquid. For example, in the first embodiment, by split addition of a cooking liquid, the alkali concentration throughout the entire cooking step can be controlled, and as a result, the total content of straight-chain fatty acid containing 24 carbon atoms, straight-chain fatty acid containing 26 carbon atoms, and straight-chain fatty acid containing 28 carbon atoms in the obtained unbleached pulp can be adjusted. Specifically, by controlling the alkali concentration of the cooking liquid, the amount of straight-chain fatty acid containing 24 carbon atoms, straight-chain fatty acid containing 26 carbon atoms, and straight-chain fatty acid containing 28 carbon atoms removed can be set within an appropriate range.

In the second embodiment, by split addition of a cooking liquid, the alkali concentration throughout the entire cooking step can be controlled, and as a result, the content of xylan or the Canadian Standard Freeness (CSF) in the obtained unbleached pulp can be adjusted. Specifically, if the alkali concentration of the cooking liquid is increased, the content of xylan can be reduced, and the Canadian Standard Freeness (CSF) can be lowered. In contrast, if the alkali concentration of the cooking liquid is decreased, the content of xylan is increased, but the Canadian Standard Freeness (CSF) can be increased.

In the third embodiment, by split addition of a cooking liquid, the alkali concentration during the entire cooking can be controlled, and as a result, the amount of fines in the obtained unbleached pulp can be adjusted. Specifically, if the alkali concentration of the cooking liquid is increased, the amount of fines is increased. In contrast, if the alkali concentration of the cooking liquid is decreased, the amount of fines is decreased. Accordingly, for example, when chips that easily generate fines are used as a pulp raw material, a prescription, in which the alkali concentration of the cooking liquid is lowered to reduce the amount of fines, etc., etc. can be adopted. In addition, the amount of fines in the obtained unbleached pulp can be adjusted by controlling the cooking time and the cooking temperature. Specifically, if the cooking time is shortened and the cooking temperature is increased, the amount of fines is increased. In contrast, if the cooking time is lengthened and the cooking temperature is lowered, the amount of fines is decreased. Therefore, for example, when chips that easily generate fines are used as a pulp raw material, a method of lengthening the cooking time and lowering the cooking temperature, etc. can be adopted.

In the fourth embodiment, by split addition of a cooking liquid, the alkali concentration throughout the entire cooking step can be controlled, and as a result, the length-weighted average fiber length of the obtained unbleached pulp can be adjusted. Specifically, if the alkali concentration of the cooking liquid is increased, the length-weighted average fiber length is shortened. In contrast, if the alkali concentration of the cooking liquid is decreased, the length-weighted average fiber length is lengthened. Accordingly, for example, when chips having a relatively short length-weighted average fiber length are used as a pulp raw material, a prescription, in which the alkali concentration of the cooking liquid is lowered to lengthen the length-weighted average fiber length, etc., can be adopted. In addition, the length-weighted average fiber length in the obtained unbleached pulp can be adjusted by controlling the cooking time and the cooking temperature.

Specifically, if the cooking time is shortened and the cooking temperature is increased, the length-weighted average fiber length is shortened. In contrast, if the cooking time is lengthened and the cooking temperature is lowered, the length-weighted average fiber length is lengthened. Therefore, for example, when chips having a relatively short length-weighted average fiber length are used as a pulp raw material, a method of lengthening the cooking time and lowering the cooking temperature, can be adopted.

(Oxygen delignification step)

In the oxygen delignification step, an alkaline chemical and oxygen are added to an unbleached pulp, and the lignin is decomposed at a high temperature and a high pressure, so that it is eluted in the alkaline chemical. The amount of bleaching chemicals used in the subsequent multi-stage bleaching steps can be reduced by delignification in the oxygen delignification step, and a reduction in the pulp quality can be minimized. As an oxygen delignification method, a known medium concentration method or high concentration method can be applied as is, and the medium concentration method, which is currently widely used and is performed at a pulp concentration of 8% to 15% by mass, is preferable.

In the oxygen delignification step performed by the medium concentration method, caustic soda or oxidized kraft white liquor can be used as alkali, and as oxygen gas, oxygen from a cryogenic separation method, oxygen from PSA (Pressure Swing Adsorption), or oxygen from VSA (Vacuum Swing Adsorption) can be used. Such oxygen gas and alkali are added to a medium-concentration pulp slurry in a medium concentration mixer, and after thorough mixing, a mixture of a pulp, oxygen and alkali is sent to a reaction tower capable of retaining the mixture under increased pressure for a certain period of time, and it is then delignified. The oxygen gas addition percentage is preferably 0.5% to 3% by mass per absolute dry pulp mass, the alkali addition percentage is preferably 0.5% to 4% by mass, the reaction temperature is preferably 80°C to 120°C, the reaction time is preferably 15 to 100 minutes, and the pulp concentration is preferably 8% to 15% by mass. As other conditions, known conditions can be applied. In the first embodiment, by appropriately controlling the alkali concentration in the oxygen delignification step, the total content of straight-chain fatty acid containing 24 carbon atoms, straight-chain fatty acid containing 26 carbon atoms, and straight-chain fatty acid containing 28 carbon atoms in the pulp can be thereby adjusted. Specifically, when the alkali concentration is increased, the amount of straight-chain fatty acid containing 24 carbon atoms, straight-chain fatty acid containing 26 carbon atoms, and straight-chain fatty acid containing 28 carbon atoms removed is increased. In contrast, when the alkali concentration is decreased, the amount of straight-chain fatty acid containing 24 carbon atoms, straight-chain fatty acid containing 26 carbon atoms, and straight-chain fatty acid containing 28 carbon atoms removed is decreased. Furthermore, when caustic soda is used as alkali, the amount of straight-chain fatty acid containing 24 carbon atoms, straight-chain fatty acid containing 26 carbon atoms, and straight-chain fatty acid containing 28 carbon atoms removed is increased. In contrast, when oxidized kraft white liquor is used as an alkali, the amount of straight-chain fatty acid containing 24 carbon atoms, straight-chain fatty acid containing 26 carbon atoms, and straight-chain fatty acid containing 28 carbon atoms removed is decreased.

In the second embodiment, by appropriately controlling the alkali concentration in the oxygen delignification step, the content of xylan and the Canadian Standard Freeness (CSF) in the obtained pulp can be thereby adjusted. Specifically, when the alkali concentration is increased, the content of xylan is decreased, and the Canadian Standard Freeness (CSF) can be lowered. In contrast, when the alkali concentration is decreased, the content of xylan is increased, but the Canadian Standard Freeness (CSF) can be increased. Furthermore, when caustic soda is used as alkali, the content of xylan is increased, but the Canadian Standard Freeness (CSF) can be increased. In contrast, when oxidized kraft white liquor is used as an alkali, the content of xylan is decreased, but the Canadian Standard Freeness (CSF) is also decreased.

In the third embodiment, by appropriately controlling the alkali concentration in the oxygen delignification step, the amount of fines in the obtained pulp can be thereby adjusted. Specifically, when the alkali concentration is increased, the amount of fines is increased. In contrast, when the alkali concentration is decreased, the amount of fines can be decreased. Furthermore, when caustic soda is used as alkali, the amount of fines can be decreased. In contrast, when oxidized kraft white liquor is used as an alkali, the amount of fines is increased.

In the fourth embodiment, by appropriately controlling the alkali concentration in the oxygen delignification step, the length-weighted average fiber length of the obtained pulp can be adjusted. Specifically, when the alkali concentration is increased, the length-weighted average fiber length becomes short. In contrast, when the alkali concentration is decreased, the length-weighted average fiber length can be lengthened. Furthermore, when caustic soda is used as alkali, the length-weighted average fiber length can be lengthened. In contrast, when oxidized kraft white liquor is used as an alkali, the length-weighted average fiber length becomes short.

(Bleaching step)

In the bleaching step, the unbleached pulp that has been subjected to oxygen delignification is bleached to obtain a bleached pulp. The bleaching method is not particularly limited, and bleaching can be carried out by a known bleaching method. Examples of the bleaching method may include an ozone bleaching stage (Z), a chlorine dioxide bleaching stage (D), an alkali extraction stage (E), an oxygen bleaching stage (O), a hydrogen peroxide bleaching stage (E/P), a peracid bleaching stage (PA), an acid washing stage (a), and an acid treatment stage (A). In the present embodiment, for example, the bleaching is preferably carried out in the order of D0-E/P-D1. It is to be noted that the Z stage is preferably not performed because it causes great damage to the pulp fibers and the stiffness of the fibers is lost, which deteriorates the defibration properties of the pulp sheet for fluff pulp.

The bleaching conditions in the D0 stage and the D1 stage are not particularly limited, and the chlorine dioxide addition percentage is preferably 0.05% to 5.0% by mass, the bleaching temperature is preferably 30°C to 90°C, the bleaching time is preferably 1 to 240 minutes, the pulp concentration is preferably 5% to 15% by mass, and pH at the time of termination of the reaction is preferably pH 2 to 6. In addition, the bleaching conditions in the E/P stage are not particularly limited, and the addition percentage of hydrogen peroxide is preferably 0.05% to 5.0% by mass, the bleaching temperature is preferably 30°C to 90°C, the bleaching time is preferably 1 to 240 minutes, the pulp concentration is preferably 5% to 15% by mass, and the pH at the time of termination of the reaction is preferably pH 10 to 12.

(Prehydrolysis step)

A prehydrolysis step can be provided before the cooking step. In the prehydrolysis step, a hardwood or a softwood is heated in the presence of water to perform a prehydrolysis treatment. Herein, the amount of water relative to the hardwood or the softwood (liquid ratio: the amount of the water (parts by mass)/the amount of the hardwood or the softwood (parts by mass)) is preferably 1.0 to 10, more preferably 1.5 to 8.0, further preferably 2.0 to 7.0, and still further preferably 2.7 to 5.0. When the liquid ratio is 1.0 or more, it is preferable because the hydrolysis proceeds sufficiently and the reaction becomes uniform. When the liquid ratio is 10 or less, it is preferable because the amount of heat required to heat up to a desired temperature is reduced, making it economical. The method of adding water is not particularly limited, and water may be added from the outside, or water originally contained in the hardwood or the softwood may be used, or when the steam is used for heating, water contained in the steam may be utilized. In addition, an alkali, an acid, a chelating agent, etc., which are chemical products that directly or indirectly aid in the hydrolysis of polysaccharides, can be added together with water.

(Cold caustic extraction step)

The bleached pulp is further treated with an alkali, so that a bleached pulp can be obtained after the cold alkali treatment. The treatment method is not particularly limited, and caustic soda can be used as alkali. The caustic soda addition percentage is preferably 5.0% to 20.0%, the bleaching temperature is preferably 20°C to 50°C, the bleaching time is preferably 1 to 240 minutes, and the pulp concentration is preferably 5% to 15% by mass.

(Sheet formation step)

In the sheet formation step, the bleached pulp obtained through the bleaching step is first dispersed in water to prepare a slurry. The pulp concentration in the slurry is preferably 0.01% to 10% by mass. In addition, if additives are added as necessary, it is preferable to add the additives to the slurry.

The sheet formation step includes a step of making a paper from the slurry using a papermaking machine. The papermaking machine used in the papermaking step is not particularly limited, and for example, papermaking machines such as a fourdrinier papermaking machine, a cylinder papermaking machine, a hybrid former and a gap former, can be used. In addition, when making a paper, the paper may be made in a single layer, or multiple layers may be combined together to make a paper.

(Dewatering and washing step)

A dewatering and washing step may be appropriately provided between various types of steps described above. In the dewatering and washing step, the raw material pulp is diluted, and is washed by combining it with dehydration. The washing method is not particularly limited, and the washing can be performed using a known washing machine. For example, a replacement washing method, a dilution/dehydration method, a press washing method, or a combination of these washing methods, can be used.

In the dewatering and washing step, the amount of straight-chain fatty acid containing 24 carbon atoms, straight-chain fatty acid containing 26 carbon atoms, and straight-chain fatty acid containing 28 carbon atoms removed during the washing can be changed by appropriately changing the ratio of filtered water/clean water used as washing water in a washing machine. The filtrated water means water that is circulated and used as washing water, and the clean water means normal water that is not circulated. In the first embodiment, specifically, by increasing the ratio of the filtered water, the amount of straight-chain fatty acid containing 24 carbon atoms, straight-chain fatty acid containing 26 carbon atoms,

and straight-chain fatty acid containing 28 carbon atoms removed can be reduced, and by increasing the ratio of the clean water, the amount of straight-chain fatty acid containing 24 carbon atoms, straight-chain fatty acid containing 26 carbon atoms, and straight-chain fatty acid containing 28 carbon atoms removed can be increased. Thus, for example, when chips with relatively low contents of carbon atoms straight-chain fatty acid containing 24 carbon atoms, straight-chain fatty acid containing 26 carbon atoms, and straight-chain fatty acid containing 28 carbon atoms are used as a pulp raw material, a method, in which the ratio of the filtered water in the washing water is increased so that straight-chain fatty acid containing 24 carbon atoms, straight-chain fatty acid containing 26 carbon atoms, and straight-chain fatty acid containing 28 carbon atoms are not removed as much, etc. can be adopted. In addition, by increasing the temperature of the washing water, the amount of straight-chain fatty acid containing 24 carbon atoms, straight-chain fatty acid containing 26 carbon atoms, and straight-chain fatty acid containing 28 carbon atoms removed can be increased, and by decreasing the temperature of the washing water, the amount of straight-chain fatty acid containing 24 carbon atoms, straight-chain fatty acid containing 26 carbon atoms, and straight-chain fatty acid containing 28 carbon atoms removed can be reduced.

In the third embodiment, in the dewatering and washing step, the amount of fines removed during the washing can be changed by appropriately changing the ratio of filtered water/clean water used as washing water in a washing machine. The filtrated water means water that is circulated and used as washing water, and the clean water means normal water that is not circulated. Specifically, by increasing the ratio of the filtered water, the amount of fines removed can be reduced, and by increasing the ratio of the clean water, the amount of fines removed can be increased. Thus, for example, when chips that easily generate fines are used as a pulp raw material, a method, in which the ratio of the filtered water in the washing water is decreased so that fines are removed, etc. can be adopted.

Moreover, in the fourth embodiment, in the dewatering and washing step, the length-weighted average fiber length can be adjusted by appropriately changing the ratio of filtered water/clean water used as washing water in a washing machine. The filtrated water means water that is circulated and used as washing water, and the clean water means normal water that is not circulated. Specifically, by increasing the ratio of the filtered water, the amount of fines removed can be reduced, and the weighted average fiber length can be relatively shortened. By increasing the ratio of the clean water, the amount of fines removed can be increased, and the weighted average fiber length can be relatively lengthened. Thus, for example, when chips having a short length-weighted average fiber length are used as a pulp raw material, a method, in which the ratio of the filtered water in the washing water is decreased and fines are removed, etc. can be adopted.

(Fiber classification step)

A fiber classification step may be appropriately provided between various types of steps described above. In a step of removing fines, a raw material pulp is diluted and passed through a screen to classify the fibers. In particular, in the third embodiment, it is preferable to classify the fibers using a metal mesh or a metal screen before the sheet formation step. By providing such a classification step, the fines pass through the mesh or the screen, and the fines can be removed. Also, the fines that have passed through the mesh by the above-described method are recovered, and the recovered fines are then added to that in which the amount of fines intends to be increased, so that the amount of fines can be increased. For example, in a laboratory, fines can be removed and recovered by washing the pulp with water on a stainless steel sieve or by using a sieving device (for example, a sieving tester that is in accordance with JIS P 8207: 2009 or TAPPI T233 cm-95 (for example, a Bauer-McNett sieving tester)). In actual production, fines can be removed and recovered by using a fiber classifier.

(Fluff mat)

The present embodiment relates to a fluff mat formed from the aforementioned pulp sheet for fluff pulp. The fluff mat in the present description is obtained by defibrating a fluff pulp obtained by defibrating the aforementioned pulp sheet for fluff pulp, and then molding the defibrated fluff pulp into a sheet shape. The fluff mat of the present embodiment has excellent water absorption properties, and can retain a liquid after it has absorbed the liquid. For this reason, the liquid backflow percentage is reduced in the fluff mat of the present embodiment.

The conditions for defibrating the pulp sheet for fluff pulp are not particularly limited, and for example, a known defibrating machine or grinding machine can be used.

The fluff mat is formed by stacking the fluff pulp under an air flow. Specifically, the defibrated fluff pulp is transported by air and is then stacked on a metal net or on a porous substrate such as a tissue or a nonwoven fabric.

The density of the fluff mat is preferably 0.058 $g/cm^3$ or more, and more preferably 0.060 $g/cm^3$ or more. The density of the resulting fluff mat is preferably 0.150 $g/cm^3$ or less. By setting the density of the fluff mat to be within the above-described range, it is possible to make the thickness of the fluff mat thin.

(Absorbent article)

The present embodiment may relate to an absorbent article having the aforementioned fluff mat. Examples of the absorbent article may include disposable diapers, incontinence pads, light incontinence pads, and sanitary napkins. The fluff mat of the present embodiment can neutralize, in particular, acidic urine.

Examples

Hereinafter, the features of the present invention will be more specifically described with reference to Examples and Comparative Examples. The materials, used amounts, proportions, treatment content, treatment procedures, and the like shown in the following Examples can be appropriately changed to the extent that such changes do not depart from the spirit of the present invention. Therefore, the scope of the present invention should not be construed as being limited by the following specific examples.

[Preparation of softwood pulp]

[Cooking]

After the chips of genus Pinus had been mixed with the chips of genus Pseudotsuga at an absolute dry mass ratio of 30 : 70, 200 g (absolute dry mass) of the mixed chips were collected and placed in a 2.5-L autoclave, and kraft cooking was then carried out under the conditions of: a liquid ratio of 5, a degree of sulfidation of a cooking liquid of 28%, an alkali addition percentage of 22%, a cooking temperature of 165°C, and a cooking time of 120 minutes. Thereafter, a black liquor and a pulp were separated from each other, and the pulp was refined using a flat screen equipped with a 10-cut screen plate to obtain an unbleached pulp with a kappa number of about 30.

[Oxygen delignification]

The unbleached pulp (70.0 g) was collected at an absolute dry mass, and caustic soda was then added to the unbleached pulp, so that the amount of the caustic soda became 2.0% by mass with respect to the absolute dry pulp mass. Subsequently, the obtained mixture was diluted with ion exchange water to adjust the pulp concentration to 10% by mass. This reaction mixture was placed in a heat-type autoclave, and 99.9% commercially available compressed oxygen gas was then injected into the autoclave to set the gauge pressure at 0.5 MPa. Thereafter, oxygen delignification was carried out at 95°C for 60 minutes. The obtained pulp was diluted with ion exchange water to 3% by mass, and was then dehydrated and washed in a Buchner funnel.

[Bleaching]

The kraft pulp (60 g) after alkaline oxygen delignification was collected at an absolute dry mass, and was then placed in a plastic bag. The pulp concentration was adjusted to 10% by mass using ion exchange water, and chlorine dioxide was added to the pulp so that the amount of chlorine dioxide became 2.0% by mass with respect to the absolute dry pulp mass. This reaction mixture was immersed in a thermostatic water bath at 70°C for 60 minutes to carry out a D0 stage treatment. Thereafter, the obtained pulp was diluted with ion exchange water to 3% by mass, and was then dehydrated and washed in a Buchner funnel.

The pulp after the D0 stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.2% by mass with respect to the absolute dry pulp mass, followed by fully mixing. Thereafter, the reaction mixture was immersed in a thermostatic water bath at 70°C for 90 minutes to carry out an E/P stage treatment. The obtained pulp was diluted with ion exchange water to 3% by mass, and was then dehydrated and washed in a Buchner funnel.

The pulp after the E/P stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.3% by mass with respect to the absolute dry pulp mass, followed by fully mixing. Thereafter, the reaction mixture was immersed in a thermostatic water bath at 70°C for 90 minutes to carry out a P stage treatment. The obtained pulp was diluted with ion exchange water to 3% by mass, and was then dehydrated and washed in a Buchner funnel.

The pulp after the P stage was placed in a plastic bag, and the pulp concentration was adjusted to 10% by mass by addition of ion exchange water. Thereafter, chlorine dioxide was added to the pulp, so that the amount of the chlorine dioxide became 0.2% by mass with respect to the absolute dry pulp mass, and the reaction mixture was then immersed in a thermostatic water bath at 70°C for 60 minutes to carry out a D1 stage bleaching. The obtained bleached softwood pulp was diluted with ion exchange water to 3% by mass, and was then dehydrated and washed in a Buchner funnel.

[Preparation of hardwood pulp]

[Cooking]

After the chips of genus Eucalyptus had been mixed with the chips of genus Acacia (Acacia mangium) at an absolute dry mass ratio of 30 : 70, 200 g (absolute dry mass) of the mixed chips were collected and placed in a 2.5-L autoclave, and kraft cooking was then carried out under the conditions of: a liquid ratio of 5, a degree of sulfidation of a cooking liquid of 28%, an alkali addition percentage of 20%, a cooking temperature of 155°C, and a cooking time of 120 minutes. Thereafter, a black liquor and a pulp were separated from each other, and the pulp was refined using a flat screen equipped with an 8-cut screen plate to obtain an unbleached pulp with a kappa number of about 18.

[Oxygen delignification]

The unbleached pulp (70.0 g) was collected at an absolute dry mass, and caustic soda was then added to the unbleached pulp, so that the amount of the caustic soda became 2.0% by mass with respect to the absolute dry pulp mass. Subsequently, the obtained mixture was diluted with ion exchange water to adjust the pulp concentration to 10% by mass. This reaction mixture was placed in a heat-type autoclave, and 99.9% commercially available compressed oxygen gas was then injected into the autoclave to set the gauge pressure at 0.5 MPa. Thereafter, oxygen delignification was carried out at 95°C for 60 minutes. The obtained pulp was diluted with ion exchange water to 3% by mass, and was then dehydrated and washed in a Buchner funnel.

[Bleaching]

The kraft pulp (60 g) after the alkaline oxygen delignification was collected at an absolute dry mass, and was placed in a plastic bag. The pulp concentration was adjusted to 10% by mass with ion exchange water, and chlorine dioxide was then added thereto so that the amount of the chlorine dioxide became 1.0% by mass with respect to the absolute dry pulp mass. This reaction mixture was immersed in a thermostatic water bath at 70°C for 60 minutes to perform a D0 stage treatment. The obtained pulp was diluted with ion exchange water to 3% by mass, and was then dehydrated and washed in a Buchner funnel.

The pulp after the D0 stage was placed in a plastic bag, and the pulp concentration was adjusted to 10% by mass by addition of ion exchange water. Thereafter, caustic soda and hydrogen peroxide were added to the pulp, so that the amount of the caustic soda became 1.0% by mass and the amount of the hydrogen peroxide became 0.2% by mass with respect to the absolute dry pulp mass, followed by fully mixing. Thereafter, the reaction mixture was immersed in a thermostatic water bath at 70°C for 90 minutes to carry out an E/P stage treatment. The obtained pulp was diluted with ion exchange water to 3% by mass, and was then dehydrated and washed in a Buchner funnel.

The pulp after the E/P stage was placed in a plastic bag, and the pulp concentration was adjusted to 10% by mass by addition of ion exchange water. Thereafter, chlorine dioxide was added to the pulp, so that the amount of the chlorine dioxide became 0.2% by mass with respect to the absolute dry pulp mass, and the reaction mixture was then immersed in a thermostatic water bath at 70°C for 60 minutes to carry out a D1 stage bleaching. The obtained bleached hardwood pulp was diluted with ion exchange water to 3% by mass, and was then dehydrated and washed in a Buchner funnel.

(Example 1)

The bleached softwood pulp and the bleached hardwood pulp after the D1 stage were mixed with each other at an absolute dry mass ratio of 0 : 100, and the mixed pulp was diluted with ion exchange water to a concentration of about 0.5% by mass, and then, using a standard circular hand papermaking machine (manufactured by KUMAGAI RIKI KOGYO Co., Ltd.), the reaction mixture was subjected to papermaking, so that the basis weight became 270 g/m$^2$, thereby obtaining a pulp sheet for fluff pulp.

(Example 2)

A pulp sheet for fluff pulp was obtained by performing the same operations as those in Example 1, with the exception that the absolute dry mass ratio between the bleached softwood pulp and the bleached hardwood pulp was set to be 20 : 80 during the papermaking.

(Example 3)

A pulp sheet for fluff pulp was obtained by performing the same operations as those in Example 1, with the exception that the absolute dry mass ratio between the bleached softwood pulp and the bleached hardwood pulp was set to be 40 : 60 during the papermaking.

(Example 4)

A pulp sheet for fluff pulp was obtained by performing the same operations as those in Example 1, with the exception that the absolute dry mass ratio between the bleached softwood pulp and the bleached hardwood pulp was set to be 60 : 40 during the papermaking.

(Example 5)

A pulp sheet for fluff pulp was obtained by performing the same operations as those in Example 1, with the exceptions that the alkali addition percentage was set to be 18% so that the kappa number of the unbleached pulp used in the preparation of the bleached hardwood pulp became 25, that the chlorine dioxide addition percentage in the D0 stage was changed to 2.0% by mass, and that the absolute dry mass ratio between the bleached softwood pulp and the bleached hardwood pulp was set to be 20 : 80 during the papermaking.

(Example 6)

A pulp sheet for fluff pulp was obtained by performing the same operations as those in Example 1, with the exceptions that the alkali addition percentage was set to be 22% so that the kappa number of the unbleached pulp used in the preparation of the bleached hardwood pulp became 13, that the chlorine dioxide addition percentage in the D0 stage was changed to 0.7% by mass, and that the absolute dry mass ratio between the bleached softwood pulp and the bleached hardwood pulp was set to be 20 : 80 during the papermaking.

(Example 7)

A pulp sheet for fluff pulp was obtained by performing the same operations as those in Example 1, with the exceptions that the chlorine dioxide addition percentage in the D0 stage upon the preparation of the bleached hardwood pulp was set to be 0.8% by mass, that the chlorine dioxide addition percentage in the D0 stage upon the preparation of the bleached softwood pulp was set to be 1.3% by mass, and that the absolute dry mass ratio between the bleached softwood pulp and the bleached hardwood pulp was set to be 20 : 80 during the papermaking.

(Example 8)

A pulp sheet for fluff pulp was obtained by performing the same operations as those in Example 1, with the exceptions that the chlorine dioxide addition percentage in the D0 stage upon the preparation of the bleached hardwood pulp was set to be 1.7% by mass, that the chlorine dioxide addition percentage in the D0 stage upon the preparation of the bleached softwood pulp was set to be 2.2% by mass, and that the absolute dry mass ratio between the bleached softwood pulp and the bleached hardwood pulp was set to be 20 : 80 during the papermaking.

(Example 9)

A pulp sheet for fluff pulp was obtained by performing the same operations as those in Example 1, with the exceptions that the absolute dry mass ratio between the bleached softwood pulp and the bleached hardwood pulp was set to be 20 : 80 during the papermaking, that the mixed pulp was diluted to a concentration of about 0.5% by mass, and that a softener (FS-8010, manufactured by SEIKO PMC CORPORATION, active ingredient: 90% by mass) was then added to the reaction mixture, so that the amount of the active ingredient became 0.1% by mass with respect to the pulp absolute dry mass ratio.

(Example 10)

A pulp sheet for fluff pulp was obtained by performing the same operations as those in Example 9, with the exception that the additive amount of the softener was changed so that the amount of the active ingredient became 0.3% by mass with respect to the pulp absolute dry mass ratio.

(Comparative Example 1)

A pulp sheet for fluff pulp was obtained by performing the same operations as those in Example 1, with the exception that the absolute dry mass ratio between the bleached softwood pulp and the bleached hardwood pulp was set to be 80 : 20 during the papermaking.

(Comparative Example 2)

A pulp sheet for fluff pulp was obtained by performing the same operations as those in Example 1, with the exceptions that the pulp after the oxygen delignification upon the preparation of the bleached hardwood pulp was washed with 10 L of boiled water (90°C), that the concentration obtained by the dilution with ion exchange water after the D0 stage, E/P stage, and D1 stage treatments was set to be 5% by mass, and that the absolute dry mass ratio between the bleached softwood pulp and the bleached hardwood pulp was set to be 20 : 80 during the papermaking

(Comparative Example 3)

A pulp sheet for fluff pulp was obtained by performing the same operations as those in Example 1, with the exceptions that the hardwood of genus Acacia was set to be Acacia crassicarpa, and that the absolute dry mass ratio between the bleached softwood pulp and the bleached hardwood pulp was set to be 20 : 80 during the papermaking.

(Comparative Example 4)

A pulp sheet for fluff pulp was obtained by performing the same operations as those in Example 1, with the exception that the absolute dry mass ratio between the bleached softwood pulp and the bleached hardwood pulp was set to be 100 : 0 during the papermaking.

(Measurement/Evaluation)

[ISO brightness]

The ISO brightness was measured in accordance with JIS P 8148: 2018.

[Viscosity]

The Viscosity was measured in accordance with JIS P 8215: 1998.

[Kappa number]

The kappa number was measured in accordance with JIS P 8211: 2011.

[C24, C26, and C28 straight-chain fatty acids]

The pulp sheet for fluff pulp was dried, about 200 mg of the dried pulp was precisely weighed, and 0.1 ml of concentrated hydrochloric acid (12 N) and 2 ml of chloroform were added thereto, followed by performing an ultrasonic treatment for 10 minutes. The obtained extract was filtrated though a membrane filter (pore diameter: 0.2 $\mu$m). Using the above-described filtrate, an analysis was carried out according to high performance liquid chromatography. As an eluent, here was used methanol : 0.1% by mass of trifluoroacetic acid-containing acetone = 50 : 50. In addition, the flow rate of the high performance liquid chromatography was set to be 1 ml/min. As a column to be used, X Bridge C18, 250 mm x 4.6 mm I.D, manufactured by Waters, was used, the temperature was set to be 30°C, and 2 $\mu$l of the filtrate was injected. As a detector, a charged particle detector was used to detect straight-chain fatty acids (higher fatty acids: $C_{23}H_{47}COOH$, $C_{25}H_{51}COOH$, and $C_{27}H_{55}COOH$), and the concentration of each fatty acid in the sample was obtained using the following calculation formula, and the total concentration was then calculated. The peak area value of each component is the area surrounded by the baseline and each peak, but for partially overlapping peaks, the peak area was separated and was calculated using the perpendicular line method of JIS K 0124: 2011.

Concentration in sample (ppm) = reference material concentration (100 ppm)/ reference material peak area value $\times$ sample peak area value $\times$ 2 (ml)/(sample mass (mg)/1000)  <Calculation formula>

[Appearance (dirts)]

The number of impurities mixed in the pulp sheet for fluff pulp was counted by visual observation. Regarding impurities, the measurement was performed for impurities with a size of 0.05 mm$^2$ or more, as specified in the impurity measurement chart described in JIS P 8208: 1998. Impurities were counted for a total of five pulp sheets for fluff pulp, and the number of impurities per 100 g of absolute dry pulp was calculated as the number of dirts.

[Defibration properties]

The defibration properties of the pulp sheet for fluff pulp were evaluated under an environment of 23°C and a relative humidity of 50%. The pulp sheet for fluff pulp, which had been humidity-conditioned under an environment of 23°C and a relative humidity of 50%, was cut into a 10 mm x 10 mm square, and 3 g thereof was weighed out and was put into a Wonder Blender (manufactured by OSAKA CHEMICAL Co., Ltd., model: WB-1, rotation number: 25000 rpm) without deviation. The pulp was defibrated for a predetermined time in 5-second intervals to obtain a cotton-like pulp (hereinafter, the pulp made into a cotton-like form by defibration is called "fluff pulp"). Subsequently, the fluff pulp was immersed in water and was gently stirred with a stick, and then, using a standard circular hand papermaking machine, a handmade paper with a basis weight of 25 g/m$^2$ for use in evaluation of defibration properties was prepared. The handmade paper for use in evaluation of defibration properties was visually observed, and the defibration time (sec) required for the undefibrated material to disappear was used as an indicator for evaluation of the defibration properties.

[Production of fluff mat]

The fluff mat was produced under an environment of 23°C and a relative humidity of 50%, using a fluff mat-producing device 100 shown in Figure 1. Herein, a pulp defibrating machine 5 (manufactured by Maruto Seiki), a stainless steel sieve 22 with a sieve opening of 180 μm (manufactured by AS ONE CORPORATION, inner diameter 75 mm x inner height 20 mm), a stainless steel sieve 24 with a sieve opening of 150 μm (manufactured by AS ONE CORPORATION, inner diameter 75 mm x inner height 20 mm), a column 20 prepared using a PET resin plate (manufactured by Acrysunday Co., Ltd., Sunday PET, thickness 0.5 mm) cut into 70 cm x 40 cm, a polypropylene powder funnel 10 (manufactured by AS ONE CORPORATION, model No. 166, aperture: 180 mm, foot outer diameter: 43 mm, foot length: 48 mm), a polypropylene powder funnel 30 (manufactured by AS ONE CORPORATION, model No. 169, aperture: 120 mm, foot outer diameter: 30 mm, foot length: 27 mm), and a household vacuum cleaner 40 (manufactured by Hitachi Home & Life Solutions, Inc., model: CV-CF4) were used to assemble the fluff mat-producing device 100 having the configuration as shown in Figure 1. First, the total weight of the stainless steel sieve with a sieve opening of 180 μm and the column was measured in advance. Next, the pulp defibrating machine and the household vacuum cleaner were started up. The suction power of the household vacuum cleaner was set to be low (suction power of approximately 80 W), and the fluff pulp without undefibrated materials obtained during the evaluation in [Defibration properties] was added little by little from the top of the fluff mat-producing device. The weight of the fluff mat formed on the stainless steel sieve with a sieve opening of 180 μm was measured from time to time, and the fluff mat was formed until the weight reached 200 g/m$^2$. After the weight had reached 200 g/m$^2$, the pulp defibrating machine and the household vacuum cleaner were stopped. After removing the column, the surface of the fluff mat was lightly pressed with a circular stainless steel plate to shape it, and the fluff mat (diameter: 75 mm) was gently removed from the sieve with a sieve opening of 180 μm without losing its shape. The surface that had been in contact with the stainless steel mesh was defined to be the wire surface of the fluff mat.

[Measurement of backflow percentage of fluff mat]

The backflow percentage of the fluff mat was measured under an environment of 23°C and a relative humidity of 50%. The produced 200 g/m$^2$ fluff mat was placed on a 5 mm thick acrylic plate with the wire side facing up. After a 2 mm thick spacer had been inserted therebetween, another 5 mm thick acrylic plate was further placed on the top, and was pressed with a tabletop pressing machine for 10 minutes. After completion of the pressing, the acrylic plate placed on the top was removed, and the resultant was then left at rest for 30 minutes. A stainless steel base plate (5 mm thick, 11 x 11 cm) was placed on a horizontal laboratory table, and two fluff mats were then placed and laminated on the top of it with the wire side facing up (the total basis weight of the fluff mats: 400 g/m$^2$). An 11 cm x 11 cm square core wrap tissue (Oji Nepia Co., Ltd., basis weight: 16 g/m$^2$) was placed on the top of the fluff mats, so that the paper flow direction became a longitudinal direction. On the top thereof, a stainless steel-made strike-through plate (10 cm x 10 cm, thickness: 85 mm, weight: 665 g) with a 25 mm diameter hole in the center thereof was placed, and after 10 seconds, 20 g of artificial urine was poured into the hole of the strike-through plate. Immediately after the artificial urine had been completely absorbed, the strike-through plate was removed. Next, an 11 cm x 11 cm square polyethylene sheet was placed on the top of the core wrap tissue, and an 11 cm x 11 cm square sponge rubber sheet made of chloroprene rubber (manufactured by AS ONE CORPORATION, thickness: 10 mm, hardness (Asker C hardness scale): 45 degrees) was placed on the top of the polyethylene sheet, and a 2.2 kg weight was placed on the top thereof. Since the total weight of the polyethylene sheet, the sponge rubber sheet, the two acrylic plates, and the 2.2 kg weight was 2.31 kg, and the area of the fluff mat was 0.00442 $m^2$, the pressure applied to the fluff mat was 5.1 kPa. The mat was pressurized at a pressure of 5.1 kPa for 3 minutes. Subsequently, the polyethylene sheet, the sponge rubber sheet, the two acrylic plates, and the 2.2 kg weight were removed, and 10 sheets of filter papers (11 cm x 11 cm square, manufactured by ADVANTEC, No. 1640) that had been humidity-conditioned under an environment of 23°C and a relative humidity of 50% and had a known weight were placed on the mat, so that the front side of the filter paper was allowed to come into contact with the core wrap tissue for disposable diapers. Then, a clear file, a urethane rubber sheet, two acrylic plates, and a 2.2 kg weight were placed on the mat again, and the filter papers were allowed to absorb the artificial urine while applying pressure for 2 minutes. After 2 minutes, the 10 filter papers were removed and weighed, and the backflow percentage was calculated using the formula described below. The artificial urine used herein was prepared by the following procedures. First, methylene blue (manufactured by Kanto Chemical Co., Inc.) was dissolved in ion exchange water to prepare a and diluted to a methylene blue aqueous solution with a concentration of 0.50% by mass. Next, 19.40 g of urea (manufactured by Kanto Chemical Co., Inc.), 8.040 g of sodium chloride (manufactured by Kanto Chemical Co., Inc.), 2.045 g of magnesium sulfate heptahydrate (manufactured by Kanto Chemical Co., Inc.), and 0.835 g of calcium chloride dihydrate were successively weighed out in a 500 ml beaker, and after adding an appropriate amount of ion exchange water, 1.50 g of 0.50 mass % methylene blue solution was added dropwise thereto. Subsequently, the resulting solution was transferred into a 1 L measuring flask and made up to 1 L to prepare artificial urine.

[Expression 6]

$$\frac{(\text{Weight [g] of 10 filter papers after liquid absorption}) - (\text{Weight [g] of 10 filter papers before liquid absorption})}{20\ [\text{g}]} \times 100\ [\%]$$

[Table 1]

| | Needle leaf tree | | Broad leaf tree | | Needle-leaf tree pulp | Broad-leaf tree pulp | Broad-leaf tree kappa number | Amount of straight-chain fatty acids with C24, C26 and C28 | Pulp sheet for fluff pulp | | | | Fluffing suitability | Fluff mat |
| | Pinus | Pseudotsuga | Eucalyptus | Acacia | | | | | ISO brightness | Viscosity | Kappa number | Dusts | Defibration properties | Backflow percentage |
| | mass % | mass % | mass % | mass % | mass % | mass % | - | ppm | % | mPa·s | - | dusts/100 g | sec | % |
| Example 1 | - | - | 30 | 70 | 0 | 100 | 1.4 | 780 | 85.6 | 8.4 | 1.4 | 4 | 25 | 66 |
| Example 2 | 30 | 70 | 30 | 70 | 20 | 80 | 1,4 | 650 | 84.5 | 11.8 | 1.2 | 8 | 20 | 70 |
| Example 3 | 30 | 70 | 30 | 70 | 40 | 60 | 1.4 | 482 | 85.2 | 12.2 | 1.0 | 4 | 20 | 74 |
| Example 4 | 30 | 70 | 30 | 70 | 60 | 40 | 1.4 | 350 | 85.6 | 11.7 | 0.8 | 0 | 15 | 79 |
| Example 5 | 30 | 70 | 30 | 70 | 20 | 80 | 1.7 | 974 | 84.5 | 14.4 | 1.2 | 8 | 15 | 72 |
| Example 6 | 30 | 70 | 30 | 70 | 20 | 80 | 1.5 | 756 | 85.6 | 7.2 | 1.1 | 4 | 25 | 74 |
| Example 7 | 30 | 70 | 30 | 70 | 20 | 80 | 1.6 | 590 | 80.9 | 13.1 | 1.3 | 0 | 20 | 72 |
| Example 8 | 30 | 70 | 30 | 70 | 20 | 80 | 1.2 | 542 | 88.4 | 9.2 | 1.0 | 4 | 20 | 73 |
| Example 9 | 30 | 70 | 30 | 70 | 20 | 80 | 1.4 | 644 | 84.5 | 11.8 | 1.3 | 4 | 15 | 70 |

| | Needle leaf tree | | Broad leaf tree | | Pulp sheet for fluff pulp | | | | | | | | Fluffing suitability | Fluff mat |
|---|---|---|---|---|---|---|---|---|---|---|---|---|---|---|
| | Pinus | Pseudotsuga | Eucalyptus | Acacia | Needle-leaf tree pulp | Broad-leaf tree pulp | Broad-leaf tree kappa number | Amount of straight-chain fatty acids with C24, C26 and C28 | ISO brightness | Viscosity | Kappa number | Dusts | Defibration properties | Backflow percentage |
| | mass % | mass % | mass % | mass % | mass % | mass % | - | ppm | % | mPa · s | - | dusts/100 g | sec | % |
| Example 10 | 30 | 70 | 30 | 70 | 20 | 80 | 1.3 | 649 | 84.5 | 11.8 | 1.3 | 4 | 15 | 70 |
| Comp. Ex. 1 | 30 | 70 | 30 | 70 | 80 | 20 | 1.3 | 280 | 84.3 | 12.5 | 0.4 | 0 | 15 | 83 |
| Comp. Ex. 2 | 30 | 70 | 30 | 70 | 20 | 80 | 1.6 | 1105 | 84.6 | 9.5 | 1.1 | 20 | 20 | 70 |
| Comp. Ex. 3 | 30 | 70 | 30 | 70 | 20 | 80 | 1.4 | 103 | 85.6 | 11.3 | 1.0 | 0 | 20 | 81 |
| Comp. Ex. 4 | 30 | 70 | - | - | 100 | 0 | - | 9 | 84.0 | 11.0 | 0.3 | 0 | 15 | 88 |

EP 4 678 813 A1

Compared to the comparative examples, in the examples, pulp sheets for fluff pulp with good appearance could be obtained, and fluff mats with suppressed backflow percentages could be obtained. In addition, the pulp sheets for fluff pulp obtained in the examples had good defibration properties.

(Example 21)

The bleached softwood pulp and the bleached hardwood pulp after the D1 stage were mixed with each other at an absolute dry mass ratio of 0 : 100, and the mixed pulp was diluted with ion exchange water to a concentration of about 0.5% by mass, and then, using a standard circular hand papermaking machine (manufactured by KUMAGAI RIKI KOGYO Co., Ltd.), the reaction mixture was subjected to papermaking, so that the basis weight became 270 g/m$^2$, thereby obtaining a pulp sheet for fluff pulp.

(Example 22)

A pulp sheet for fluff pulp was obtained by performing the same operations as those in Example 21, with the exception that the absolute dry mass ratio between the bleached softwood pulp and the bleached hardwood pulp was set to be 40 : 60 during the papermaking.

(Example 23)

A pulp sheet for fluff pulp was obtained by performing the same operations as those in Example 21, with the exception that the absolute dry mass ratio between the bleached softwood pulp and the bleached hardwood pulp was set to be 60 : 40 during the papermaking.

(Example 24)

A pulp sheet for fluff pulp was obtained by performing the same operations as those in Example 21, with the exceptions that the absolute dry mass ratio between genus Eucalyptus and genus Acacia (Acacia mangium) as a hardwood was set to be 0 : 100, and that the absolute dry mass ratio between the bleached softwood pulp and the bleached hardwood pulp was set to be 0:100 during the papermaking.

(Example 25)

A pulp sheet for fluff pulp was obtained by performing the same operations as those in Example 21, with the exceptions that the absolute dry mass ratio between genus Eucalyptus and genus Acacia (Acacia mangium) as a hardwood was set to be 100 : 0, and that the absolute dry mass ratio between the bleached softwood pulp and the bleached hardwood pulp was set to be 0:100 during the papermaking.

(Example 26)

In Example 21, the absolute dry mass ratio between genus Eucalyptus and genus Acacia (Acacia mangium) as a hardwood was set to be 100 : 0, the bleached hardwood pulp after the D1 stage was placed in a plastic bag, caustic soda was added to the pulp, so that the amount of the caustic soda became 7.5% by mass with respect to the absolute dry pulp mass. Ion exchange water was used to adjust the pulp concentration to 10% by mass, and the reaction mixture was immersed in a thermostatic water bath at 40°C for 60 minutes to carry out a cold alkali treatment. The bleached hardwood pulp obtained after the cold alkali treatment was diluted with ion exchange water to 3% by mass, and was then dehydrated and washed in a Buchner funnel. The bleached hardwood pulp after the cold alkali treatment was diluted to a concentration of about 0.5% by mass, and then, using a standard circular hand papermaking machine (manufactured by KUMAGAI RIKI KOGYO Co., Ltd.), the reaction mixture was subjected to papermaking, so that the basis weight became 270 g/m$^2$, thereby obtaining a pulp sheet for fluff pulp. Except for the above-described operations, the same operations as those in Example 21 were carried out.

(Example 27)

A pulp sheet for fluff pulp was obtained by performing the same operations as those in Example 21, with the exceptions that the pulp was diluted to a concentration of about 0.5% by mass during the papermaking, and that a softener (FS-8010, manufactured by SEIKO PMC CORPORATION, active ingredient: 90% by mass) was then added to the reaction mixture, so that the amount of the active ingredient became 0.1% by mass with respect to the pulp absolute dry mass ratio.

(Example 28)

A pulp sheet for fluff pulp was obtained by performing the same operations as those in Example 21, with the exception that the pulp was diluted to a concentration of about 0.5% by mass during the papermaking, and that the softener was then added so that the amount of the active ingredient became 0.3% by mass with respect to the pulp absolute dry mass ratio.

(Comparative Example 21)

A pulp sheet for fluff pulp was obtained by performing the same operations as those in Example 21, with the exception that the absolute dry mass ratio between the bleached softwood pulp and the bleached hardwood pulp was set to be 100 : 0 during the papermaking.

(Comparative Example 22)

A pulp sheet for fluff pulp was obtained by performing the same operations as those in Example 21, with the exception that the pulp was dehydrated during the papermaking so that the pulp concentration became 10% by mass, and was beaten at 10,000 rotations in a PFI mill (manufactured by KUMAGAI RIKI KOGYO Co., Ltd.) and was then diluted to a concentration of about 0.5% by mass.

(Comparative Example 23)

A pulp sheet for fluff pulp was obtained by performing the same operations as those in Example 21, with the exception that genus Acacia as a hardwood was changed to Acacia mearnsii.

(Measurement/Evaluation)

[Amount of xylan]

According to an NREL/TP-510-42618 method, the pulp sheet for fluff pulp was subjected to acid hydrolysis using sulfuric acid, and was then subjected to quantitative analysis of xylose using ion chromatography. Using the obtained data, the amount (%) of xylan was calculated using the following formula:

Amount of xylan (g) = amount of xylose measured ($\mu$M) $\times$ 132 (molecular weight of xylose unit)/overdecomposition percentage of sugar by acid (%) Amount of xylan (%) = amount of xylan (g)/mass of pulp sheet for fluff pulp subjected to ion chromatography (g)

[Permanganate number]

The Permanganate number was measured in accordance with JIS P 8206: 1982.

[Canadian Standard Freeness]

In accordance with JIS P 8220-1: 2012, the pulp sheet for fluff pulp was disintegrated to obtain a disintegrated pulp. The Canadian Standard Freeness (CSF) of the disintegrated pulp was measured in accordance with JIS P 8121-2: 2012.

[ISO brightness]

The ISO brightness was measured in accordance with JIS P 8148: 2018.

[Kappa number]

The kappa number was measured in accordance with JIS P 8211: 2011.

[Changed amount in brightness]

The pulp sheet for fluff pulp was subjected to a heat treatment at 105°C in accordance with JIS P 8154-1: 2008. The heat treatment time was set to be for 72 hours. After the heat treatment, the ISO brightness of the pulp sheet for fluff pulp was measured in accordance with JIS P 8148: 2018. The change amount in the ISO brightness was calculated using the following equation:

$$\text{Change amount of ISO brightness } (\%) = B_0 \, (\%) - B_1 \, (\%)$$

In the above equation, $B_0$ indicates the ISO brightness of the pulp sheet for fluff pulp before the heat treatment, and $B_1$ indicates the ISO brightness of the pulp sheet for fluff pulp after the heat treatment.

[Defibration properties]

The defibration properties of the pulp sheet for fluff pulp were evaluated under an environment of 23°C and a relative humidity of 50%. The pulp sheet for fluff pulp, which had been humidity-conditioned under an environment of 23°C and a relative humidity of 50%, was cut into a 10 mm x 10 mm square, and 3 g thereof was weighed out and was put into a Wonder Blender (manufactured by OSAKA CHEMICAL Co., Ltd., model: WB-1, rotation number: 25000 rpm) without deviation. The pulp was defibrated for a predetermined time in 5-second intervals to obtain a cotton-like pulp (hereinafter, the pulp made into a cotton-like form by defibration is called "fluff pulp"). Subsequently, the fluff pulp was immersed in water and was gently stirred with a stick, and then, using a standard circular hand papermaking machine, a handmade paper with a basis weight of 25 g/m$^2$ for use in evaluation of defibration properties was prepared. The handmade paper for use in evaluation of defibration properties was visually observed, and the defibration time (sec) required for the undefibrated material to disappear was used as an indicator for evaluation of the defibration properties.

[Production of fluff mat]

A fluff mat was produced by the same method as that in the aforementioned [Production of fluff mat].

[Measurement of backflow percentage of fluff mat]

The backflow percentage of the fluff mat was measured by the same method as that in the aforementioned [Measurement of backflow percentage of fluff mat].

[Table 2]

| | Needle leaf tree | | Broad leaf tree | | Pulp sheet for fluff pulp | | | | | | | | Fading property | Fluffing suitability | Fluff mat |
|---|---|---|---|---|---|---|---|---|---|---|---|---|---|---|---|
| | Pinus | Pseudotsuga | Eucalyptus | Acacia | Needle-leaf tree pulp | Broad-leaf tree pulp | Broad-leaf tree pulp kappa number | Xylan amount | K value | Freeness | ISO brightness | Kappa number | ISO brightness change amount | Defibration properties | Backflow percentage |
| | mass % | mass % | mass % | mass % | mass % | mass % | - | % | | mL | % | - | % | sec | % |
| Example 21 | 30 | 70 | 30 | 70 | 20 | 80 | 1.4 | 16.0 | 1.2 | 493 | 84.5 | 1.3 | 3.5 | 20 | 70 |
| Example 22 | 30 | 70 | 30 | 70 | 40 | 60 | 1.3 | 13.4 | 1.0 | 552 | 85.2 | 1.1 | 3.3 | 15 | 74 |
| Example 23 | 30 | 70 | 30 | 70 | 60 | 40 | 1.5 | 10.2 | 0.7 | 612 | 85.6 | 0.8 | 2.6 | 15 | 79 |
| Example 24 | - | - | 0 | 100 | 0 | 100 | 1.5 | 17.2 | 1.3 | 430 | 86.2 | 1.5 | 4.1 | 25 | 64 |
| Example 25 | - | - | 100 | 0 | 0 | 100 | 1.7 | 17.0 | 1.5 | 470 | 85.4 | 1.6 | 4.2 | 20 | 65 |
| Example 26 | - | - | 100 | 0 | 0 | 100 | 0.3 | 3.0 | 0.2 | 465 | 90.2 | 0.3 | 1.6 | 20 | 65 |
| Example 27 | 30 | 70 | 30 | 70 | 20 | 80 | 1.5 | 15.3 | 1.2 | 525 | 84.5 | 1.3 | 3.5 | 15 | 70 |
| Example 28 | 30 | 70 | 30 | 70 | 20 | 80 | 1.5 | 15.1 | 1.3 | 544 | 84.3 | 1.4 | 4.0 | 15 | 70 |
| Comp. Ex. 21 | 30 | 70 | - | - | 100 | 0 | - | 2.1 | 1.5 | 730 | 84.3 | 1.6 | 1.9 | 15 | 88 |
| Comp. Ex. 22 | 30 | 70 | 30 | 70 | 20 | 80 | 1.7 | 14.3 | 1.5 | 350 | 84.5 | 1.5 | 3.5 | 70 | 68 |
| Comp. Ex. 23 | 30 | 70 | 30 | 70 | 20 | 80 | 2.8 | 21.3 | 1.8 | 486 | 84.6 | 1.9 | 6.0 | 40 | 70 |

27

Compared to the comparative examples, in the examples, pulp sheets for fluff pulp with high brightness and excellent defibration properties could be obtained. In addition, in the examples, fluff mats with suppressed backflow percentage could be obtained.

(Example 31)

The bleached softwood pulp and the bleached hardwood pulp after the D1 stage were mixed with each other at an absolute dry mass ratio of 20 : 80, and the mixed pulp was diluted with ion exchange water to a concentration of about 0.5% by mass, and then, using a standard circular hand papermaking machine (manufactured by KUMAGAI RIKI KOGYO Co., Ltd.), the reaction mixture was subjected to papermaking, so that the basis weight became 270 g/m$^2$, thereby obtaining a pulp sheet for fluff pulp.

(Example 32)

A pulp sheet for fluff pulp was obtained by performing the same operations as those in Example 31, with the exception that the absolute dry mass ratio between the bleached softwood pulp and the bleached hardwood pulp was set to be 60 : 40 during the papermaking.

(Example 33)

A pulp sheet for fluff pulp was obtained by performing the same operations as those in Example 31, with the exceptions that the kappa number of the unbleached pulp during the preparation of the bleached hardwood pulp was set to be 13, and that the chlorine dioxide addition percentage in the D0 stage was set to be 0.7% by mass.

(Example 34)

A pulp sheet for fluff pulp was obtained by performing the same operations as those in Example 31, with the exception that the absolute dry mass ratio between the bleached softwood pulp and the bleached hardwood pulp was set to be 0 : 100 during the papermaking.

(Example 35)

In Example 34, 20 g of the bleached hardwood pulp after the D1 stage was collected at an absolute dry weight, and was then placed on a 80-mesh stainless steel sieve, and the pulp was stirred by hand, while showering with ion exchange water, so as to remove the fines. At this time, the amount of the ion exchange water was set to be 20 L. The same operation was repeated until the amount of the pulp required for papermaking was secured. The pulp remaining on the stainless steel sieve after the operations was subjected to papermaking. A pulp sheet for fluff pulp was obtained by performing the same operations as those in Example 34 with the aforementioned exceptions.

(Example 36)

In Example 34, 20 g of the bleached hardwood pulp after the D1 stage was collected at an absolute dry weight, and was then placed on a 60-mesh stainless steel sieve, and the pulp was stirred by hand, while showering with ion exchange water, so as to remove the fines. At this time, the amount of the ion exchange water was set to be 100 L. The same operation was repeated until the amount of the pulp required for papermaking was secured. The pulp remaining on the stainless steel sieve after the operations was subjected to papermaking. A pulp sheet for fluff pulp was obtained by performing the same operations as those in Example 34 with the aforementioned exceptions.

(Example 37)

A pulp sheet for fluff pulp was obtained by performing the same operations as those in Example 31, with the exceptions that the absolute dry mass ratio between the bleached softwood pulp and the bleached hardwood pulp was set to be 20 : 80 during the papermaking, that the pulp was diluted to a concentration of about 0.5% by mass, and that a softener (FS-8010, manufactured by SEIKO PMC CORPORATION, active ingredient: 90% by mass) was then added to the resulting pulp, so that the amount of the active ingredient became 0.1% by mass with respect to the pulp absolute dry mass ratio.

(Example 38)

A pulp sheet for fluff pulp was obtained by performing the same operations as those in Example 31, with the exceptions that the absolute dry mass ratio between the bleached softwood pulp and the bleached hardwood pulp was set to be 20 : 80 during the papermaking, that the pulp was diluted to a concentration of about 0.5% by mass, and that a softener (FS-8010, manufactured by SEIKO PMC CORPORATION, active ingredient: 90% by mass) was then added to the resulting pulp, so that the amount of the active ingredient became 0.3% by mass with respect to the pulp absolute dry mass ratio.

(Example 39)

A pulp sheet for fluff pulp was obtained by performing the same operations as those in Example 34, with the exceptions that the pulp was diluted to a concentration of about 0.5% by mass during the papermaking, and that a softener (FS-8010, manufactured by SEIKO PMC CORPORATION, active ingredient: 90% by mass) was then added to the resulting pulp, so that the amount of the active ingredient became 0.1% by mass with respect to the pulp absolute dry mass ratio.

(Example 40)

A pulp sheet for fluff pulp was obtained by performing the same operations as those in Example 34, with the exceptions that the pulp was diluted to a concentration of about 0.5% by mass during the papermaking, and that a softener (FS-8010, manufactured by SEIKO PMC CORPORATION, active ingredient: 90% by mass) was then added to the resulting pulp, so that the amount of the active ingredient became 0.3% by mass with respect to the pulp absolute dry mass ratio.

(Comparative Example 31)

A pulp sheet for fluff pulp was obtained by performing the same operations as those in Example 31, with the exception that the absolute dry mass ratio between the bleached softwood pulp and the bleached hardwood pulp was set to be 100 : 0 during the papermaking.

(Comparative Example 32)

In Example 31, after the D1 stage, the bleached softwood pulp was mixed with the bleached hardwood pulp at an absolute dry mass ratio of 20 : 80, and 20 g of the mixed pulp was collected at an absolute dry weight. The collected pulp was placed on a 80-mesh stainless steel sieve, and the pulp was stirred by hand, while showering with ion exchange water, so as to remove the fines. At this time, the amount of the ion exchange water was set to be 20 L. The fines that passed through the sieve together with the ion exchange water were received by a 400-mesh stainless steel sieve. The resulting fines were mixed with the pulp before the fines had been removed, and the resulting pulp was then subjected to papermaking. Other than these operations, the same operations as those in Example 1 were carried out to obtain a pulp sheet for fluff pulp.

(Measurement/Evaluation)

[Amount of fines]

The percentage of fines comprised in the pulp sheet for fluff pulp was calculated as follows. First, the fiber length of the pulp fibers in the pulp sheet for fluff pulp was measured in the region of 0 to 7.60 mm, using a fiber analyzer (FS-5 manufactured by Valmet) in accordance with JIS P 8226-2: 2011. The number ($n_i$) of fibers was counted every 0.02 mm in the length range, and the length-weighted fiber length distribution was depicted. Based on Formula (2) described in JIS P 8226-2: 2011, the percentage of the number of the fibers weighted by length in the range of 0 to 0.10 mm was calculated, and the obtained value was defined to be the amount of fines (%).

[ISO brightness]

The ISO brightness was measured in accordance with JIS P 8148: 2018.

[Kappa number]

The kappa number was measured in accordance with JIS P 8211: 2011.

[Defibration properties]

The defibration time (sec) was measured by the same method as that in the aforementioned [Defibration properties], and it was then used as an indicator for evaluation of the defibration properties.

[Production of fluff mat]

A fluff mat was produced by the same method as that in the aforementioned [Production of fluff mat].

[Measurement of backflow percentage of fluff mat]

The backflow percentage of the fluff mat was measured by the same method as that in the aforementioned [Measurement of backflow percentage of fluff mat].

[Measurement of backflow percentage of diaper]

The backflow percentage of a diaper was all measured under an environment of 23°C and a relative humidity of 50%. First, a test sample simulating a diaper was prepared in a laboratory using the following procedures. A fluff mat with a basis weight of 200 g/m$^2$ was prepared using the above-described method, and was then pressed for 10 minutes with a 2 mm spacer between them. The produced 200 g/m$^2$ fluff mat was placed on a 5 mm thick acrylic plate with the wire side facing up. After a 2 mm thick spacer had been inserted therebetween, another 5 mm thick acrylic plate was further placed on the top, and was pressed with a tabletop pressing machine for 10 minutes. After completion of the pressing, the acrylic plate placed on the top was removed, and the resultant was then left at rest for 30 minutes. A stainless steel base plate (5 mm thick, 11 x 11 cm) was placed on a horizontal laboratory table, and a fluff mat was then placed on the top of it with the wire side facing up. A superabsorbent polymer (AQUAKEEP SA60SXII, manufactured by Sumitomo Seika Chemicals Co., Ltd.) was sprinkled as evenly as possible on top of the resultant to a thickness of 140 g/m$^2$. A fluff mat was then placed on the top of the resultant with the wire side facing up. An 11 cm x 11 cm square core wrap tissue (Oji Nepia Co., Ltd., basis weight: 16 g/m$^2$) was placed on the top of the fluff mats, so that the paper flow direction became a longitudinal direction. Then, a polypropylene spunbond nonwoven fabric (Oji Nepia Co., Ltd., basis weight: 21 g/m$^2$) was placed on the top of the resultant, so that the flow direction of the nonwoven fabric became the longitudinal direction. On the top thereof, a stainless steel-made strike-through plate (10 cm x 10 cm, thickness: 85 mm, weight: 665 g) with a 25 mm diameter hole in the center thereof was placed, and after 10 seconds, 20 g of artificial urine was poured into the hole of the strike-through plate. Immediately after the artificial urine had been completely absorbed, the strike-through plate was removed. Next, an 11 cm x 11 cm square polyethylene sheet was placed on the top of the polypropylene nonwoven fabric, then, an 11 cm x 11 cm square sponge rubber sheet made of chloroprene rubber (manufactured by AS ONE CORPORATION, thickness: 10 mm, hardness (Asker C hardness scale): 45 degrees) was placed on the top of the polyethylene sheet, then, an 11 cm x 11 cm square, 5 mm thick acrylic plate was placed on the top thereof, and then, a 2.2 kg weight was placed on the top thereof. Since the total weight of the polyethylene sheet, the sponge rubber sheet, the two acrylic plates, and the 2.2 kg weight was 2.31 kg, and the area of the fluff mat was 0.00442 m$^2$, the pressure applied to the fluff mat was 5.1 kPa. The mat was pressurized at a pressure of 5.1 kPa for 3 minutes. Subsequently, the polyethylene sheet, the sponge rubber sheet, the two acrylic plates, and the 2.2 kg weight were removed, and 10 sheets of filter papers (11 cm x 11 cm square, manufactured by ADVANTEC, No. 1640) that had been humidity-conditioned under an environment of 23°C and a relative humidity of 50% and had a known weight were placed on the mat, so that the front side of the filter paper was allowed to come into contact with the polypropylene nonwoven fabric. Then, a polyethylene sheet, a sponge rubber sheet, acrylic plates, and a 2.2 kg weight were placed on the mat again, and the filter papers were allowed to absorb the artificial urine while applying pressure for 2 minutes. After 2 minutes, the 10 filter papers were removed and weighed, and the backflow percentage was calculated using the following formula.

[Expression 7]

$$\frac{\text{(Weight [g] of 10 filter papers after liquid absorption)} - \text{(Weight [g] of 10 filter papers before liquid absorption)}}{20 \text{ [g]}} \times 100 \text{ [\%]}$$

[Table 3]

| | Needle leaf tree | | Broad leaf tree | | Pulp sheet for fluff pulp | | | | | | Fluff mat | Fluffing suitability | Diaper |
| | Pinus | Pseudotsuga | Eucalyptus | Acacia | Needle-leaf tree pulp | Broad-leaf tree pulp | Broad-leaf tree pulp kappa number | Ultrafine fiber amount | ISO brightness | Kappa number | Backflow percentage | Defibration properties | Backflow amount |
| | mass % | mass % | mass % | mass % | mass % | mass % | - | % | % | - | % | sec | % |
|---|---|---|---|---|---|---|---|---|---|---|---|---|---|
| Example 31 | 30 | 70 | 30 | 70 | 20 | 80 | 1.4 | 7.6 | 84.5 | 1.2 | 70 | 20 | 4 |
| Example 32 | 30 | 70 | 30 | 70 | 60 | 40 | 1.4 | 6.2 | 85.2 | 1.0 | 79 | 15 | 6 |
| Example 33 | 30 | 70 | 30 | 70 | 20 | 80 | 1.1 | 9.5 | 85.6 | 0.7 | 69 | 25 | 4 |
| Example 34 | - | - | 30 | 70 | 0 | 100 | 1.3 | 8.1 | 86.2 | 1.3 | 64 | 25 | 4 |
| Example 35 | - | - | 30 | 70 | 0 | 100 | 1.5 | 5.2 | 85.4 | 1.5 | 66 | 20 | 4 |
| Example 36 | - | - | 30 | 70 | 0 | 100 | 1.4 | 2.2 | 83.8 | 1.4 | 70 | 15 | 4 |
| Example 37 | 30 | 70 | 30 | 70 | 20 | 80 | 1.7 | 7.6 | 82.2 | 1.3 | 70 | 15 | 4 |
| Example 38 | 30 | 70 | 30 | 70 | 20 | 80 | 1,6 | 7.4 | 82.2 | 1.3 | 70 | 15 | 4 |
| Example 39 | - | - | 30 | 70 | 0 | 100 | 1.5 | 7.9 | 85.4 | 1.5 | 64 | 20 | 4 |
| Example 40 | - | - | 30 | 70 | 0 | 100 | 1.5 | 8.0 | 85.4 | 1.5 | 64 | 15 | 4 |
| Comp. Ex. 31 | 30 | 70 | 30 | 70 | 100 | 0 | - | 8.4 | 84.5 | 0.3 | 88 | 15 | 25 |

| | Needle leaf tree | | Broad leaf tree | | Pulp sheet for fluff pulp | | | | | | Fluff mat | Fluffing suitability | Diaper |
|---|---|---|---|---|---|---|---|---|---|---|---|---|---|
| | Pinus | Pseudotsuga | Eucalyptus | Acacia | Needle-leaf tree pulp | Broad-leaf tree pulp | Broad-leaf tree pulp kappa number | Ultrafine fiber amount | ISO brightness | Kappa number | Backflow percentage | Defibration properties | Backflow amount |
| | mass % | mass % | mass % | mass % | mass % | mass % | - | % | % | - | % | sec | % |
| Comp. Ex. 32 | 30 | 70 | 30 | 70 | 20 | 80 | 1.6 | 11.5 | 84.3 | 1.3 | 69 | 40 | 4 |

EP 4 678 813 A1

Compared to the comparative examples, in the examples, pulp sheets for fluff pulp with good defibration properties could be obtained, and fluff mats with suppressed backflow percentage could be obtained.

(Example 41)

The bleached softwood pulp and the bleached hardwood pulp after the D1 stage were mixed with each other at an absolute dry mass ratio of 0 : 100, the mixed pulp was diluted with ion exchange water to a concentration of about 0.5% by mass, and thereafter, using a standard circular hand papermaking machine (manufactured by KUMAGAI RIKI KOGYO Co., Ltd.), the resulting pulp was subjected to papermaking to a basis weight of 270 g/m$^2$, thereby obtaining a pulp sheet for fluff pulp.

(Example 42)

A pulp sheet for fluff pulp was obtained by performing the same operations as those in Example 41, with the exception that the absolute dry mass ratio between the chips of genus Eucalyptus and the chips of genus Acacia (Acacia mangium) used in the preparation of a hardwood pulp was changed to 100 : 0.

(Example 43)

A pulp sheet for fluff pulp was obtained by performing the same operations as those in Example 41, with the exception that the absolute dry mass ratio between the chips of genus Eucalyptus and the chips of genus Acacia (Acacia mangium) used in the preparation of a hardwood pulp was changed to 0 : 100.

(Example 44)

A pulp sheet for fluff pulp was obtained by performing the same operations as those in Example 41, with the exception that the absolute dry mass ratio between the bleached softwood pulp and the bleached hardwood pulp was set to be 20 : 80 during the papermaking.

(Example 45)

A pulp sheet for fluff pulp was obtained by performing the same operations as those in Example 41, with the exception that the absolute dry mass ratio between the bleached softwood pulp and the bleached hardwood pulp was set to be 40 : 60 during the papermaking.

(Example 46)

A pulp sheet for fluff pulp was obtained by performing the same operations as those in Example 41, with the exception that the absolute dry mass ratio between the bleached softwood pulp and the bleached hardwood pulp was set to be 60 : 40 during the papermaking.

(Comparative Example 41)

A pulp sheet for fluff pulp was obtained by performing the same operations as those in Example 41, with the exceptions that the absolute dry mass ratio between genus Pinus and genus Pseudotsuga as a softwood was set to be 100 : 0, and that the absolute dry mass ratio between the bleached softwood pulp and the bleached hardwood pulp was set to be 60 : 40 during the papermaking.

(Comparative Example 42)

A pulp sheet for fluff pulp was obtained by performing the same operations as those in Example 44, with the exception that the absolute dry mass ratio between the bleached softwood pulp and the bleached hardwood pulp was set to be 100 : 0 during the papermaking.

(Measurement/Evaluation)

[Length-weighted average fiber length]

The disintegrated average fiber length (length-weighted average fiber length) of pulp fibers comprised in the pulp sheet for fluff pulp was measured. The length-weighted fiber length was measured using a fiber analyzer (FS-5 manufactured by Valmet) in accordance with JIS P 8226-2: 2011.

[Fiber coarseness]

In accordance with JIS P 8220-1: 2012, the pulp sheet for fluff pulp was disintegrated, and the fiber coarseness was then measured using a fiber analyzer (FS-5 manufactured by Valmet).

[ISO brightness]

The ISO brightness was measured in accordance with JIS P 8148: 2018.

[Kappa number]

The kappa number was measured in accordance with JIS P 8211: 2011.

[Fluffing of pulp]

The pulp was fluffed under an environment of 23°C and a relative humidity of 50%. The pulp sheet for fluff pulp, which had been humidity-conditioned under an environment of 23°C and a relative humidity of 50%, was cut into a 10 mm x 10 mm square, and 3 g thereof was weighed out and was put into a Wonder Blender (manufactured by OSAKA CHEMICAL Co., Ltd., model: WB-1, rotation number: 25000 rpm) without deviation. The pulp was defibrated for a predetermined time in 5-second intervals. After confirming the defibration time required for the undefibrated material to disappear, the pulp was converted to a cotton-like state for that defibration time. Hereinafter, the pulp converted to a cotton-like state by defibration is referred to as a "fluff pulp."

[Production of fluff mat]

The fluff mat was produced under an environment of 23°C and a relative humidity of 50%, using a fluff mat-producing device 100 shown in Figure 1. Herein, a pulp defibrating machine 5 (manufactured by Maruto Seiki), a stainless steel sieve 22 with a sieve opening of 180 $\mu$m (manufactured by AS ONE CORPORATION, inner diameter 75 mm x inner height 20 mm), a stainless steel sieve 24 with a sieve opening of 150 $\mu$m (manufactured by AS ONE CORPORATION, inner diameter 75 mm x inner height 20 mm), a column 20 prepared using a PET resin plate (manufactured by Acrysunday Co., Ltd., Sunday PET, thickness 0.5 mm) cut into 70 cm x 40 cm, a polypropylene powder funnel 10 (manufactured by AS ONE CORPORATION, model No. 166, aperture: 180 mm, foot outer diameter: 43 mm, foot length: 48 mm), a polypropylene powder funnel 30 (manufactured by AS ONE CORPORATION, model No. 169, aperture: 120 mm, foot outer diameter: 30 mm, foot length: 27 mm), and a household vacuum cleaner 40 (manufactured by Hitachi Home & Life Solutions, Inc., model: CV-CF4) were used to assemble the fluff mat-producing device 100 having the configuration as shown in Figure 1. First, the total weight of the stainless steel sieve with a sieve opening of 180 $\mu$m and the column was measured in advance. Next, the pulp defibrating machine and the household vacuum cleaner were started up. The suction power of the household vacuum cleaner was set to be low (suction power of approximately 80 W), and the fluff pulp without undefibrated materials obtained in [Fluffing of pulp] was added little by little from the top of the fluff mat-producing device. The weight of the fluff mat formed on the stainless steel sieve with a sieve opening of 180 $\mu$m was measured from time to time, and the fluff mat was formed until the weight reached 200 g/m². After the weight had reached 200 g/m², the pulp defibrating machine and the household vacuum cleaner were stopped. After removing the column, the surface of the fluff mat was lightly pressed with a circular stainless steel plate to shape it, and the fluff mat (diameter: 75 mm) was gently removed from the sieve with a sieve opening of 180 $\mu$m without losing its shape. It is to be noted that the surface that had been in contact with the stainless steel mesh was defined to be the wire surface of the fluff mat.

[Density of fluff mat]

The density of the fluff mat was calculated by measuring the basis weight and thickness of the fluff mat. The following operations were all carried out under an environment of 23°C and a relative humidity of 50%. The produced 200 g/m² fluff mat was placed on a 5 mm thick acrylic plate with the wire side facing up. After a 2 mm thick spacer had been inserted therebetween, another 5 mm thick acrylic plate was placed on the top and was then pressed with a tabletop press for 10 minutes. After completion of the pressing, the acrylic plate placed on the top was removed, and the resultant was left at rest for 30 minutes. Subsequently, the thickness was measured with the wire side facing up, using a thickness gauge (manufactured by TECLOCK Co., Ltd., model: PG-14A, pressure load: 0.394 N, gauge diameter: 16 mm) at the pressure specified in JIS L 1086: 2007. Then, the basis weight was calculated by measuring the weight of the produced fluff mat with a diameter of 75 mm using an electronic balance.

[Measurement of backflow percentage of fluff mat]

The backflow percentage of the fluff mat was measured by the same method as that in the aforementioned [Measurement of backflow percentage of fluff mat].

[Table 4]

| | Needle leaf tree | | Broad leaf tree | | Pulp sheet for fluff pulp | | | | | | | Fluff mat | |
| | Pinus | Pseudotsuga | Eucalyptus | Acacia | Needle-leaf tree pulp | Broad-leaf tree pulp | Broad-leaf tree pulp kappa number | Fiber form | | ISO brightness | Kappa number | Mat density | Backflow percentage |
| | | | | | | | | Length-weighted average fiber length | Fiber coarseness | | | | |
| | mass % | mass % | mass % | mass % | mass % | mass % | - | mm | mg/m | % | - | g/cm³ | % |
| Example 41 | - | - | 30 | 70 | 0 | 100 | 1.3 | 0.78 | 0.043 | 84.5 | 1.3 | 0.0667 | 65 |
| Example 42 | - | - | 100 | 0 | 0 | 100 | 1.5 | 0.82 | 0.062 | 84.6 | 1.5 | 0.0658 | 66 |
| Example 43 | - | - | 0 | 100 | 0 | 100 | 1.2 | 0.71 | 0.048 | 85.6 | 1.2 | 0.0673 | 64 |
| Example 44 | 30 | 70 | 30 | 70 | 20 | 80 | 1.4 | 0.82 | 0.051 | 86.2 | 1.1 | 0.0637 | 70 |
| Example 45 | 30 | 70 | 30 | 70 | 40 | 60 | 1.7 | 1.04 | 0.082 | 86.3 | 1.2 | 0.0610 | 74 |
| Example 46 | 30 | 70 | 30 | 70 | 60 | 40 | 1.5 | 1.32 | 0.105 | 82.9 | 0.7 | 0.0585 | 79 |
| Comp. Ex. 41 | 100 | 0 | 30 | 70 | 60 | 40 | 1.2 | 1.50 | 0.152 | 85.7 | 0.7 | 0.0573 | 81 |
| Comp. Ex. 42 | 30 | 70 | - | - | 100 | 0 | - | 3.20 | 0.354 | 84.3 | 0.3 | 0.0541 | 88 |

36

Compared to the comparative examples, in the examples, fluff mats having high mat density (i.e. with thin thickness) and suppressed backflow percentage could be obtained.

Reference Signs List

| | |
|---|---|
| 5 | Pulp defibrating machine |
| 10 | Polypropylene powder funnel |
| 20 | Column |
| 22, 24 | Stainless steel sieve |
| 30 | Polypropylene powder funnel |
| 40 | Cleaner (suction machine) |
| 22 | Stainless steel sieve with sieve opening of 180 $\mu$m |
| 100 | Fluff mat-producing device |

**Claims**

1.   A pulp sheet for fluff pulp, comprising a hardwood pulp, wherein

the content of the hardwood pulp is 25% by mass or more, with respect to the total pulp components in the pulp sheet for fluff pulp, and
the total content of straight-chain fatty acid containing 24 carbon atoms, straight-chain fatty acid containing 26 carbon atoms and straight-chain fatty acid containing 28 carbon atoms in the pulp sheet for fluff pulp is 300 to 1000 ppm.

2.   The pulp sheet for fluff pulp according to claim 1, wherein the ISO brightness measured in accordance with JIS P 8148: 2018 is 80% or more.

3.   The pulp sheet for fluff pulp according to claim 1, wherein the viscosity measured in accordance with JIS P 8215: 1998 is 7.0 to 15.0 mPa·s.

4.   The pulp sheet for fluff pulp according to claim 1, wherein the kappa number of the hardwood pulp is 0.5 to 2.0.

5.   The pulp sheet for fluff pulp according to claim 1, wherein the content of the hardwood pulp is 50% by mass or more, with respect to the total pulp components in the pulp sheet for fluff pulp.

6.   The pulp sheet for fluff pulp according to claim 1, wherein the kappa number measured in accordance with JIS P 8211: 2011 is smaller than 1.5.

7.   A pulp sheet for fluff pulp, comprising a hardwood pulp, wherein

the content of xylan in the pulp sheet for fluff pulp is 20% by mass or less, and
when a disintegrated pulp obtained by disintegrating the pulp sheet for fluff pulp in accordance with JIS P 8220-1: 2012 is measured in terms of Canadian Canadian Standard Freeness (CSF) measured in accordance with JIS P 8121: 2012-2, the Canadian Standard Freeness (CSF) is 400 to 640 mL.

8.   The pulp sheet for fluff pulp according to claim 7, wherein
the ISO brightness measured in accordance with JIS P 8148: 2018 is 82% or more.

9.   The pulp sheet for fluff pulp according to claim 7, wherein

when the ISO brightness is measured in accordance with JIS P 8154-1: 2008 before and after heating the pulp sheet for fluff pulp at 105°C for 72 hours, and the ISO brightness reduction percentage is calculated according to the following equation, the brightness change amount is 5.0% or less.

$$\text{Amount (\%) of ISO brightness changed} = B_0\,(\%) - B_1\,(\%)$$

(wherein $B_0$ represents the ISO brightness of the pulp sheet for fluff pulp before the heat treatment, and $B_1$ represents the ISO brightness of the pulp sheet for fluff pulp after the heat treatment.)

10. The pulp sheet for fluff pulp according to claim 7, wherein
the kappa number of the hardwood pulp is 0.3 to 2.0.

11. The pulp sheet for fluff pulp according to claim 7, wherein the content of the hardwood pulp is 25% by mass or more, with respect to the total pulp components in the pulp sheet for fluff pulp.

12. The pulp sheet for fluff pulp according to claim 7, wherein
the kappa number measured in accordance with JIS P 8211: 2011 is smaller than 1.7.

13. The pulp sheet for fluff pulp according to claim 7, wherein the Permanganate number measured in accordance with JIS P 8206: 1982 is smaller than 1.6.

14. A pulp sheet for fluff pulp, comprising a hardwood pulp, wherein
when the length-weighted fiber length of pulp fibers comprised in the pulp sheet for fluff pulp is measured in accordance with JIS P 8226-2: 2011, the percentage of the number of the fibers weighted by length in the range of 0 to 0.1 mm in the length-weighted fiber length distribution is 10% or less

15. The pulp sheet for fluff pulp according to claim 14, wherein
the ISO brightness measured in accordance with JIS P 8148: 2018 is 82% or more.

16. The pulp sheet for fluff pulp according to claim 14, wherein
the kappa number of the hardwood pulp is 0.5 to 2.0.

17. The pulp sheet for fluff pulp according to claim 14, wherein
the kappa number measured in accordance with JIS P 8211: 2011 is smaller than 1.7.

18. The pulp sheet for fluff pulp according to claim 14, wherein
the content of the hardwood pulp is 25% by mass or more, with respect to the total pulp components in the pulp sheet for fluff pulp.

19. A pulp sheet for fluff pulp, comprising a hardwood pulp, wherein
the fiber coarseness measured by disintegrating the pulp sheet for fluff pulp in accordance with JIS P 8220-1: 2012 is 0.030 to 0.120 mg/m.

20. The pulp sheet for fluff pulp according to claim 19, wherein
the length-weighted average fiber length of pulp fibers comprised in the pulp sheet for fluff pulp, which is measured in accordance with JIS P 8226-2: 2011, is 0.65 mm or more and less than 1.50 mm.

21. The pulp sheet for fluff pulp according to claim 19, wherein
the ISO brightness measured in accordance with JIS P 8148: 2018 is 82% or more.

22. The pulp sheet for fluff pulp according to claim 19, wherein
the kappa number of the hardwood pulp is 0.5 to 2.0.

23. The pulp sheet for fluff pulp according to claim 19, wherein
the content of the hardwood pulp is 25% by mass or more, with respect to the total pulp components in the pulp sheet for fluff pulp.

24. The pulp sheet for fluff pulp according to claim 19, wherein
the kappa number measured in accordance with JIS P 8211: 2011 is smaller than 1.7.

25. A fluff mat formed from the pulp sheet for fluff pulp according to any one of claims 1 to 24.

26. The fluff mat according to claim 25, which has a density of 0.058 g/cm$^3$ or more.

27. An absorbent article, comprising the fluff mat according to claim 25.

[Figure 1]

## INTERNATIONAL SEARCH REPORT

| International application No. |
|---|
| **PCT/JP2024/004814** |

**A.    CLASSIFICATION OF SUBJECT MATTER**

*D21B 1/06*(2006.01)i; *A61F 13/53*(2006.01)i; *D21H 11/04*(2006.01)i; *D21H 15/02*(2006.01)i
FI:    D21B1/06; D21H11/04; A61F13/53 300; D21H15/02

According to International Patent Classification (IPC) or to both national classification and IPC

**B.    FIELDS SEARCHED**

Minimum documentation searched (classification system followed by classification symbols)

D21B1/00-1/38;D21C1/00-11/14;D21D1/00-99/00;D21F1/00-13/12;D21G1/00-9/00;D21H11/00-27/42;D21J1/00-7/00;
A61F13/00-13/84;A61L15/00-15/64

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | WO 2022/138870 A1 (DAIO PAPER CORPORATION) 30 June 2022 (2022-06-30) | 1-6, 14-18 |
| Y | claim 1, paragraphs [0006], [0008], [0022], [0035], [0042]-[0044], [0058], [0061]-[0063], examples 1, 10, 13, comparative example 3, tables 1-2 | 7-13, 25-27 |
| X | | 19-20, 21-27 |
| A | JP 2020-117818 A (OJI HOLDINGS CORPORATION) 06 August 2020 (2020-08-06) claim 1, paragraph [0008] | 1-6, 25-27 |
| A | JP 2015-151655 A (NIPPON PAPER INDUSTRIES CO., LTD.) 24 August 2015 (2015-08-24) | 1-6 |
| Y | claim 1, paragraphs [0007], [0013], [0015], [0041], [0045], example 1 | 7-13, 25-27 |
| A | JP 2022-546713 A (STORA ENSO OYJ) 07 November 2022 (2022-11-07) paragraph [0022] | 19-27 |
| A | JP 2017-141541 A (GP CELLULOSE GMBH) 17 August 2017 (2017-08-17) paragraphs [0036], [0052] | 19-27 |

☑ Further documents are listed in the continuation of Box C.          ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 May 2024** | **21 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2024/004814**

### C.    DOCUMENTS CONSIDERED TO BE RELEVANT

| Category* | Citation of document, with indication, where appropriate, of the relevant passages | Relevant to claim No. |
|---|---|---|
| P, X | JP 7297966 B1 (DAIO PAPER CORPORATION) 26 June 2023 (2023-06-26) <br> claims 1, 2, example 1, paragraphs [0005], [0043], [0052], [0071]-[0073], fig. 1, table 2 | 14-15, 17-18, 25-27 |

## INTERNATIONAL SEARCH REPORT

International application No.

**PCT/JP2024/004814**

| Box No. III | Observations where unity of invention is lacking (Continuation of item 3 of first sheet) |

This International Searching Authority found multiple inventions in this international application, as follows:

Document 1: WO 2022/138870 A1 (DAIO PAPER CORPORATION) 30 June 2022 (2022-06-30) claim 1, paragraph [0008], examples 1, 13

Claims are classified into the following four inventions.

(Invention 1) Claims 1-6 and claims 25-27 referring to any of claims 1-6

In addition, claims 1-6 and claims 25-27 referring to any of claims 1-6 have the special technical feature in which "the total content of straight-chain fatty acids with 24 carbon atoms, straight-chain fatty acids with 26 carbon atoms, and straight-chain fatty acids with 28 carbon atoms contained in the pulp sheet for fluff pulp is 300 to 1000 ppm." and is thus classified as invention 1.

(Invention 2) claims 7-13 and claims 25-27 which are not classified as invention 1 and referring to any of claims 7-13

Claims 7-13 and claims 25-27 which are not classified as invention 1 and referring to any of claims 7-13 share, with claim 1 classified as invention 1, the common technical feature of a "pulp sheet for fluff pulp comprising hardwood pulp." However, said technical feature does not make a contribution over the prior art in light of the disclosure of document 1 (in particular, claim 1, paragraph [0008], examples 1, 13), and thus cannot be considered a special technical feature. In addition, there are no other same as or corresponding special technical features in claim 1.

In addition, claims 7-13 and claims 25-27 which are not classified as invention 1 and referring to any of claims 7-13 are not dependent on claim 1. In addition, claims 7-13 and claims 25-27 which are not classified as invention 1 and referring to any of claims 7-13 are not substantially identical or equivalent to any of the claims classified as invention 1.

Thus, claims 7-13 and claims 25-27 which are not classified as invention 1 and referring to any of claims 7-13 cannot be classified as invention 1.

Also, claims 7-13 and claims 25-27 which are not classified as invention 1 and referring to any of claims 7-13 have the special technical feature in which "for disintegrated pulp obtained by disintegrating pulp sheets for fluff pulp in accordance with JISP8220-1: 2012, where the content of xylan contained in the pulp sheet for fluff pulp is 20 mass% or less, when the Canadian standard freeness (CSF) is measured in accordance with JISP8121: 2012-2, the Canadian standard freeness (CSF) is 400 to 640 mL," and are thus classified as invention 2.

(Invention 3) claims 14-18 and claims 25-27 which are not classified as inventions 1-2 and referring to any of claims 14-18

Claims 14-18 and claims 25-27 which are not classified as inventions 1-2 and referring to any of claims 14-18 share, with claim 1 classified as invention 1, the common technical feature of a "pulp sheet for fluff pulp comprising hardwood pulp." However, said technical feature does not make a contribution over the prior art in light of the disclosure of document 1 (in particular, claim 1, paragraph [0008], examples 1, 13), and thus cannot be considered a special technical feature. In addition, there are no other same as or corresponding special technical features in claim 1.

In addition, claims 14-18 and claims 25-27 which are not classified as inventions 1-2 and referring to any of claims 14-18 are not dependent on claim 1. In addition, claims 14-18 and claims 25-27 which are not classified as inventions 1-2 and referring to any of claims 14-18 are not substantially identical or equivalent to any of the claims classified as inventions 1-2.

Thus, claims 14-18 and claims 25-27 which are not classified as inventions 1-2 and referring to any of claims 14-18 cannot be classified as inventions 1-2.

Also, claims 14-18 and claims 25-27 which are not classified as inventions 1-2 and referring to any of claims 14-18 have the special technical feature in which "for pulp fibers contained in pulp sheets for fluff pulp, when length-weighted fiber length is measured in accordance with JISP8226-2: 2011, the proportion of the number of fibers weighted by lengths in the range of 0 to 0.1 mm in the length-weighted fiber length distribution is 10% or less" and are thus classified as invention 3.

(Invention 4) claims 19-24 and claims 25-27 which are not classified as inventions 1-3 and referring to any of claims 19-24

Claims 19-24 and claims 25-27 which are not classified as inventions 1-3 and referring to any of claims 19-24 share, with claim 1 classified as invention 1, the technical feature of a "pulp sheet for fluff pulp comprising hardwood pulp." However, said technical feature does not make a contribution over the prior art in light of the

Form PCT/ISA/210 (continuation of first sheet) (July 2022)

## INTERNATIONAL SEARCH REPORT

International application No.

**PCT/JP2024/004814**

| Box No. III | Observations where unity of invention is lacking (Continuation of item 3 of first sheet) |
|---|---|

disclosure of document 1 (in particular, claim 1, paragraph [0008], examples 1, 13), and thus cannot be considered a special technical feature. In addition, there are no other same as or corresponding special technical features in claim 1.

In addition, claims 19-24 and claims 25-27 which are not classified as inventions 1-3 and referring to any of claims 19-24 are not dependent on claim 1. In addition, claims 19-24 and claims 25-27 which are not classified as inventions 1-3 and referring to any of claims 19-24 are not substantially identical or equivalent to any of the claims classified as inventions 1-3.

Thus, claims 19-24 and claims 25-27 which are not classified as inventions 1-3 and referring to any of claims 19-24 cannot be classified as inventions 1-3.

In addition, claims 19-24 and claims 25-27 which are not classified as inventions 1-3 and referring to any of claims 19-24 have the special technical feature in which "the pulp sheet for fluff pulp was disintegrated in accordance with JISP8220-1: 2012, and the measured fiber roughness was 0.030 to 0.120 mg/m" and are thus classified as invention 4.

1. [✓] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**     [ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[✓] No protest accompanied the payment of additional search fees.

Form PCT/ISA/210 (continuation of first sheet) (July 2022)

**INTERNATIONAL SEARCH REPORT**
Information on patent family members

International application No.

**PCT/JP2024/004814**

| Patent document cited in search report | | | Publication date (day/month/year) | Patent family member(s) | | | Publication date (day/month/year) |
|---|---|---|---|---|---|---|---|
| WO | 2022/138870 | A1 | 30 June 2022 | CN | 116583642 | A | |
| JP | 2020-117818 | A | 06 August 2020 | (Family: none) | | | |
| JP | 2015-151655 | A | 24 August 2015 | (Family: none) | | | |
| JP | 2022-546713 | A | 07 November 2022 | US paragraph [0027] CN | 2022/0290376 114302991 | A1 A | |
| JP | 2017-141541 | A | 17 August 2017 | US paragraphs [0035], [0051] | 2014/0318725 | A1 | |
| JP | 7297966 | B1 | 26 June 2023 | JP | 2023-147065 | A | |

Form PCT/ISA/210 (patent family annex) (July 2022)

**EP 4 678 813 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012211411 A
- WO 2022138870 A